(19) **Europäisches Patentamt / European Patent Office / Office européen des brevets**

(11) **EP 4 223 229 A1**

(12) **EUROPEAN PATENT APPLICATION**
published in accordance with Art. 153(4) EPC

(43) Date of publication:
**09.08.2023 Bulletin 2023/32**

(21) Application number: **21875460.4**

(22) Date of filing: **24.09.2021**

(51) International Patent Classification (IPC):
*A61B 10/00* (2006.01)   *G06T 7/00* (2017.01)
*G06N 3/04* (2023.01)   *G16H 30/20* (2018.01)
*G16H 50/20* (2018.01)   *G16H 50/50* (2018.01)
*G06N 20/10* (2019.01)   *G06N 20/20* (2019.01)
*A61B 5/055* (2006.01)

(52) Cooperative Patent Classification (CPC):
**A61B 5/055; A61B 10/00; G06N 3/04; G06N 20/10; G06N 20/20; G06T 7/00; G16H 30/20; G16H 50/20; G16H 50/50**

(86) International application number:
**PCT/JP2021/035194**

(87) International publication number:
**WO 2022/071158 (07.04.2022 Gazette 2022/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.10.2020 JP 2020167010**
**25.12.2020 JP 2020217833**

(71) Applicant: **FUJIFILM Corporation**
**Tokyo 106-8620 (JP)**

(72) Inventors:
• **LI, Yuanzhong**
  **Ashigarakami-gun, Kanagawa 258-8577 (JP)**
• **WANG, Caihua**
  **Ashigarakami-gun, Kanagawa 258-8577 (JP)**

(74) Representative: **Hughes, Andrea Michelle**
**Dehns Germany**
**Theresienstraße 6-8**
**80333 München (DE)**

(54) **DIAGNOSIS ASSISTANCE DEVICE, METHOD FOR OPERATING DIAGNOSIS ASSISTANCE DEVICE, PROGRAM FOR OPERATING DIAGNOSIS ASSISTANCE DEVICE, DEMENTIA DIAGNOSIS ASSISTANCE METHOD, AND LEARNED MODEL FOR DERIVING DEMENTIA FINDINGS**

(57) There is provided a diagnosis support device including a processor and a memory connected to or built in the processor, in which the processor is configured to: acquire a medical image; extract a plurality of anatomical regions of an organ from the medical image; input images of the plurality of anatomical regions to a plurality of feature amount derivation models prepared for each of the plurality of anatomical regions, and output a plurality of feature amounts for each of the plurality of anatomical regions from the feature amount derivation models; input the plurality of feature amounts which are output for each of the plurality of anatomical regions to a disease opinion derivation model, and output a disease opinion from the disease opinion derivation model; and present the opinion.

FIG. 3

EP 4 223 229 A1

**Description**

BACKGROUND OF THE INVENTION

1. Field of the Invention

A technique of the present disclosure relates to a diagnosis support device, an operation method of a diagnosis support device, an operation program of a diagnosis support device, a dementia diagnosis support method, and a trained dementia opinion derivation model.

2. Description of the Related Art

In diagnosis of a disease, for example, dementia represented by Alzheimer's disease, a doctor refers to a medical image such as a head magnetic resonance imaging (MRI) image. The doctor obtains a dementia opinion by observing, for example, a degree of atrophy of a hippocampus, a parahippocampal gyrus, an amygdala, and the like, a degree of a vascular disorder of a white matter, the presence or absence of a decrease in blood flow metabolism in a frontal lobe, a temporal lobe, and an occipital lobe.

JP6483890B describes a diagnosis support device that derives a dementia opinion on a head MRI image by a machine learning model and provides the dementia opinion to a doctor. The diagnosis support device described in JP6483890B extracts a plurality of anatomical regions according to a Brodmann's brain map or the like from the head MRI image, and calculates a Z value indicating a degree of atrophy of each of the anatomical regions. In addition, the calculated Z value of each of the anatomical regions is input to a machine learning model, and a dementia opinion is output from the machine learning model.

SUMMARY OF THE INVENTION

As described above, in order to obtain an opinion of a disease such as dementia, it is necessary to thoroughly examine each of anatomical regions of an organ such as a brain from various viewpoints. However, in JP6483890B, only one index value such as the Z value which is statistically obtained is used. For this reason, there is a limit to prediction accuracy of a disease opinion that is obtained with only such limited information.

In one embodiment according to the technique of the present disclosure, there are provided a diagnosis support device, an operation method of a diagnosis support device, an operation program of a diagnosis support device, a dementia diagnosis support method, and a trained dementia opinion derivation model capable of obtaining a more accurate disease opinion.

According to the present disclosure, there is provided a diagnosis support device including: a processor; and a memory connected to or built in the processor, in which the processor is configured to: acquire a medical image; extract a plurality of anatomical regions of an organ from the medical image; input images of the plurality of anatomical regions to a plurality of feature amount derivation models prepared for each of the plurality of anatomical regions, and output a plurality of feature amounts for each of the plurality of anatomical regions from the feature amount derivation models; input the plurality of feature amounts which are output for each of the plurality of anatomical regions to a disease opinion derivation model, and output a disease opinion from the disease opinion derivation model; and present the opinion.

Preferably, the feature amount derivation model includes at least one of an auto-encoder, a single-task convolutional neural network for class determination, or a multi-task convolutional neural network for class determination.

Preferably, the processor is configured to: input an image of one anatomical region of the anatomical regions to the plurality of different feature amount derivation models, and output the feature amounts from each of the plurality of feature amount derivation models.

Preferably, the processor is configured to: input disease-related information related to the disease to the disease opinion derivation model in addition to the plurality of feature amounts.

Preferably, the disease opinion derivation model is configured by any one method of a neural network, a support vector machine, or boosting.

Preferably, the processor is configured to: perform normalization processing of matching the acquired medical image with a reference medical image prior to extraction of the anatomical regions.

Preferably, the organ is a brain and the disease is dementia. In this case, preferably, the plurality of anatomical regions include at least one of a hippocampus or a temporal lobe. Further, preferably, the disease-related information includes at least one of a volume of the anatomical region, a score of a dementia test, a test result of a genetic test, a test result of a spinal fluid test, or a test result of a blood test.

According to the present disclosure, there is provided an operation method of a diagnosis support device, the method including: acquiring a medical image; extracting a plurality of anatomical regions of an organ from the medical image; inputting images of the plurality of anatomical regions to a plurality of feature amount derivation models prepared for each of the plurality of anatomical regions, and outputting a plurality of feature amounts for each of the plurality of anatomical regions from the feature amount derivation models; inputting the plurality of feature amounts which are output for each of the plurality of anatomical regions to a disease opinion derivation model, and outputting a disease opinion from the disease opinion derivation model; and presenting the opinion.

According to the present disclosure, there is provided an operation program of a diagnosis support device, the program causing a computer to execute a process including: acquiring a medical image; extracting a plurality of anatomical regions of an organ from the medical image; inputting images of the plurality of anatomical regions to a plurality of feature amount derivation models prepared for each of the plurality of anatomical regions, and outputting a plurality of feature amounts for each of the plurality of anatomical regions from the feature amount derivation models; inputting the plurality of feature amounts which are output for each of the plurality of anatomical regions to a disease opinion derivation model, and outputting a disease opinion from the disease opinion derivation model; and presenting the opinion.

According to the present disclosure, there is provided a dementia diagnosis support method causing a computer that includes a processor and a memory connected to or built in the processor to execute a process including: acquiring a medical image in which a brain appears; extracting a plurality of anatomical regions of the brain from the medical image; inputting images of the plurality of anatomical regions to a plurality of feature amount derivation models prepared for each of the plurality of anatomical regions, and outputting a plurality of feature amounts for each of the plurality of anatomical regions from the feature amount derivation models; inputting the plurality of feature amounts which are output for each of the plurality of anatomical regions to a dementia opinion derivation model, and outputting a dementia opinion from the dementia opinion derivation model; and presenting the opinion.

According to the present disclosure, there is provided a trained dementia opinion derivation model for causing a computer to execute a function of outputting a dementia opinion in response to inputting of a plurality of feature amounts, in which the plurality of feature amounts are output from a plurality of feature amount derivation models prepared for each of a plurality of anatomical regions of a brain by inputting images of the plurality of anatomical regions to the plurality of feature amount derivation models, the anatomical regions being extracted from a medical image in which a brain appears.

According to the technique of the present disclosure, it is possible to provide a diagnosis support device, an operation method of a diagnosis support device, an operation program of a diagnosis support device, a dementia diagnosis support method, and a trained dementia opinion derivation model capable of obtaining a more accurate disease opinion.

BRIEF DESCRIPTION OF THE DRAWINGS

Fig. 1 is a diagram illustrating a medical system including a diagnosis support device.
Fig. 2 is a block diagram illustrating a computer including the diagnosis support device.
Fig. 3 is a block diagram illustrating a processing unit of a CPU of the diagnosis support device.
Fig. 4 is a diagram illustrating processing of a normalization unit.
Fig. 5 is a diagram illustrating processing of an extraction unit.
Fig. 6 is a diagram illustrating processing of a feature amount derivation unit.
Fig. 7 is a diagram illustrating processing of a dementia opinion derivation unit.
Fig. 8 is a diagram illustrating a first display screen.
Fig. 9 is a diagram illustrating a second display screen.
Fig. 10 is a diagram illustrating a configuration of an auto-encoder and a structure of a feature amount derivation model.
Fig. 11 is a diagram explaining convolution processing.
Fig. 12 is a diagram illustrating a configuration of operation data.
Fig. 13 is a diagram explaining pooling processing.
Fig. 14 is a diagram illustrating an outline of processing in a learning phase of the auto-encoder.
Fig. 15 is a diagram illustrating an outline of processing in a learning phase of a dementia opinion derivation model.
Fig. 16 is a flowchart illustrating a processing procedure of the diagnosis support device.
Fig. 17 is a diagram illustrating another example of dementia opinion information.
Fig. 18 is a diagram illustrating still another example of dementia opinion information.
Fig. 19 is a diagram illustrating a configuration of a single-task convolutional neural network for class determination and a structure of a feature amount derivation model.
Fig. 20 is a diagram illustrating an outline of processing in a learning phase of the single-task convolutional neural network for class determination.

Fig. 21 is a diagram illustrating a configuration of a multi-task convolutional neural network for class determination and a structure of a feature amount derivation model.

Fig. 22 is a diagram illustrating an outline of processing in a learning phase of the multi-task convolutional neural network for class determination.

Fig. 23 is a diagram illustrating processing of a feature amount derivation unit according to a fourth embodiment.

Fig. 24 is a diagram illustrating processing of a dementia opinion derivation unit according to a fifth embodiment.

Fig. 25 is a diagram illustrating an outline of processing in a learning phase of a dementia opinion derivation model according to the fifth embodiment.

Fig. 26 is a diagram illustrating a configuration of an auto-encoder, a configuration of a single-task convolutional neural network for class determination, and a structure of a feature amount derivation model.

Fig. 27 is a diagram illustrating a detailed configuration of an output unit.

Fig. 28 is a diagram illustrating an outline of processing in a learning phase of the auto-encoder and the single-task convolutional neural network for class determination.

Fig. 29 is a graph illustrating a change of a weight given to a loss of the auto encoder.

Fig. 30 is a diagram illustrating processing of a dementia opinion derivation unit according to a sixth embodiment.

Fig. 31 is a table showing a performance comparison between a method of predicting progress of dementia that is described in literatures in the related art and a method of predicting progress of dementia according to the sixth embodiment.

DESCRIPTION OF THE PREFERRED EMBODIMENTS

[First Embodiment]

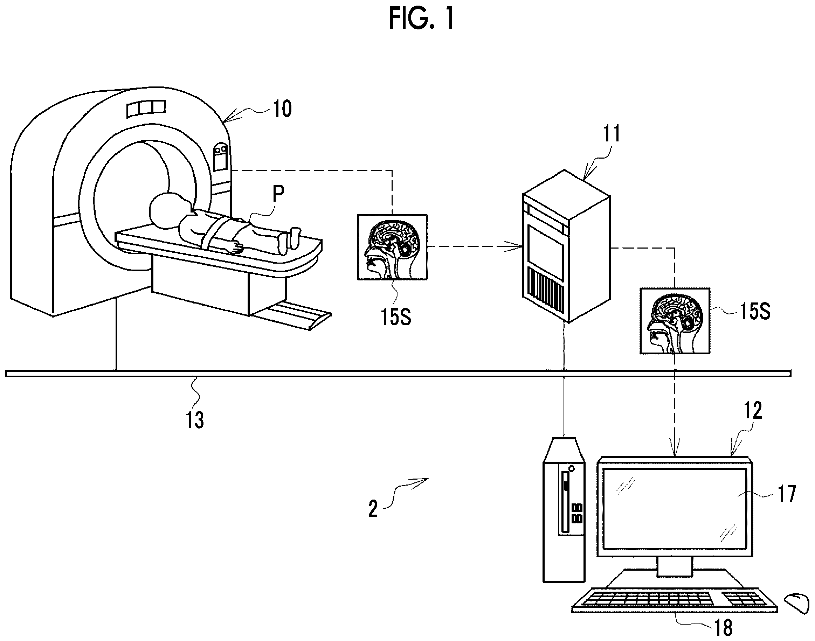

As illustrated in Fig. 1 as an example, a medical system 2 includes an MRI apparatus 10, a picture archiving and communication system (PACS) server 11, and a diagnosis support device 12. The MRI apparatus 10, the PACS server 11, and the diagnosis support device 12 are connected to a local area network (LAN) 13 provided in a medical facility, and can communicate with each other via the LAN 13.

The MRI apparatus 10 images a head of a patient P and outputs a head MRI image 15. The head MRI image 15 is voxel data representing a three-dimensional shape of the head of the patient P. In Fig. 1, a head MRI image 15S having a sagittal cross section is illustrated. The MRI apparatus 10 transmits the head MRI image 15 to the PACS server 11. The PACS server 11 stores and manages the head MRI image 15 from the MRI apparatus 10. The head MRI image 15 is an example of a "medical image" according to the technique of the present disclosure.

The diagnosis support device 12 is, for example, a desktop personal computer, and includes a display 17 and an input device 18. The input device 18 is a keyboard, a mouse, a touch panel, a microphone, or the like. A doctor transmits a distribution request of the head MRI image 15 of the patient P to the PACS server 11 by operating the input device 18. The PACS server 11 searches for the head MRI image 15 of the patient P that is requested to be distributed, and distributes the head MRI image 15 to the diagnosis support device 12. The diagnosis support device 12 displays the head MRI image 15 distributed from the PACS server 11 on the display 17. The doctor diagnoses dementia on the patient P by observing a brain of the patient P appearing in the head MRI image 15. The brain is an example of an "organ" according to the technique of the present disclosure, and the dementia is an example of a "disease" according to the technique of the present disclosure. In Fig. 1, only one MRI apparatus 10 and one diagnosis support device 12 are illustrated. On the other hand, a plurality of MRI apparatuses 10 and a plurality of diagnosis support devices 12 may be provided.

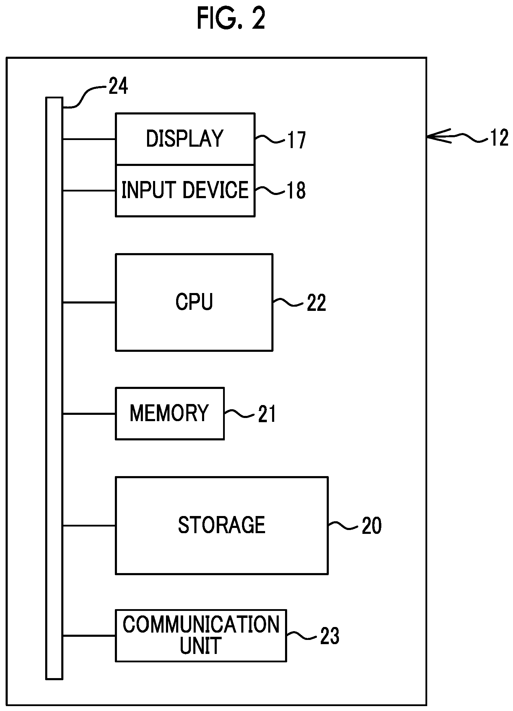

As illustrated in Fig. 2 as an example, a computer including the diagnosis support device 12 includes a storage 20, a memory 21, a central processing unit (CPU) 22, and a communication unit 23, in addition to the display 17 and the input device 18. The components are connected to each other via a bus line 24. The CPU 22 is an example of a "processor" according to the technique of the present disclosure.

The storage 20 is a hard disk drive that is built in the computer including the diagnosis support device 12 or is connected via a cable or a network. Alternatively, the storage 20 is a disk array in which a plurality of hard disk drives are connected in series. The storage 20 stores a control program such as an operating system, various application programs, and various data associated with the programs. A solid state drive may be used instead of the hard disk drive.

The memory 21 is a work memory which is necessary to execute processing by the CPU 22. The CPU 22 loads the program stored in the storage 20 into the memory 21, and executes processing according to the program. Thereby, the CPU 22 collectively controls each unit of the computer. The communication unit 23 controls transmission of various types of information to an external apparatus such as the PACS server 11. The memory 21 may be built in the CPU 22.

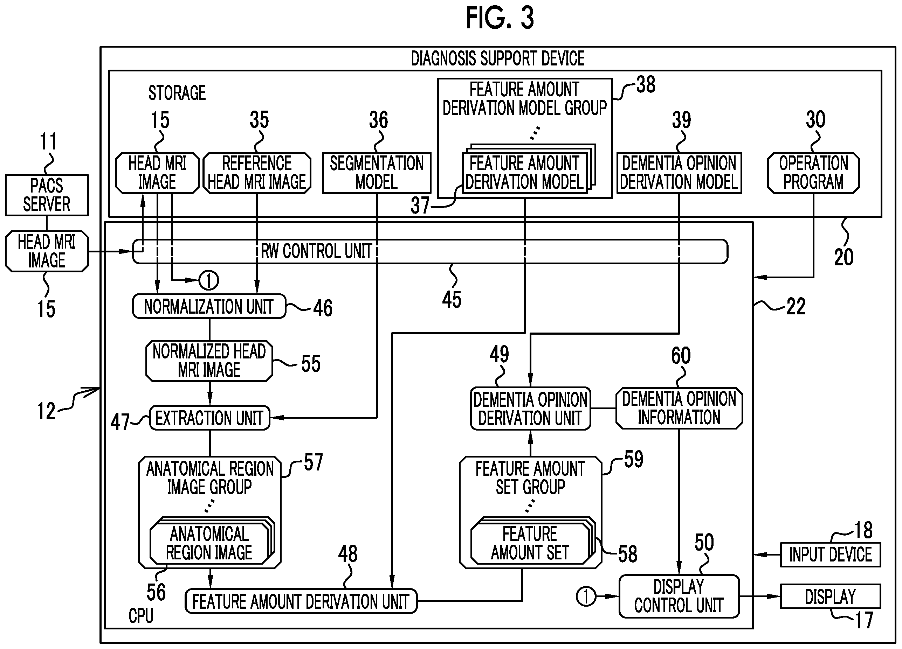

As illustrated in Fig. 3 as an example, an operation program 30 is stored in the storage 20 of the diagnosis support device 12. The operation program 30 is an application program for causing the computer to function as the diagnosis support device 12. That is, the operation program 30 is an example of "the operation program of the diagnosis support device" according to the technique of the present disclosure. The storage 20 also stores the head MRI image 15, a reference head MRI image 35, a segmentation model 36, a feature amount derivation model group 38 including a plurality of feature amount derivation models 37, and a dementia opinion derivation model 39.

In a case where the operation program 30 is started, the CPU 22 of the computer including the diagnosis support device 12 functions as a read/write (hereinafter, abbreviated as RW) control unit 45, a normalization unit 46, an extraction unit 47, a feature amount derivation unit 48, a dementia opinion derivation unit 49, and a display control unit 50, in cooperation with the memory 21 and the like.

The RW control unit 45 controls storing of various types of data in the storage 20 and reading of various types of data in the storage 20. For example, the RW control unit 45 receives the head MRI image 15 from the PACS server 11, and stores the received head MRI image 15 in the storage 20. In Fig. 3, only one head MRI image 15 is stored in the storage 20. On the other hand, a plurality of head MRI images 15 may be stored in the storage 20.

The RW control unit 45 reads the head MRI image 15 of the patient P designated by the doctor for diagnosing dementia from the storage 20, and outputs the read head MRI image 15 to the normalization unit 46 and the display control unit 50. The RW control unit 45 acquires the head MRI image 15 by reading the head MRI image 15 from the storage 20.

In addition, the RW control unit 45 reads the reference head MRI image 35 from the storage 20, and outputs the read reference head MRI image 35 to the normalization unit 46. The RW control unit 45 reads the segmentation model 36 from the storage 20, and outputs the read segmentation model 36 to the extraction unit 47. The RW control unit 45 reads the feature amount derivation model group 38 from the storage 20, and outputs the read feature amount derivation model group 38 to the feature amount derivation unit 48. Further, the RW control unit 45 reads the dementia opinion derivation model 39 from the storage 20, and outputs the read dementia opinion derivation model 39 to the dementia opinion derivation unit 49.

The normalization unit 46 performs normalization processing of matching the head MRI image 15 with the reference head MRI image 35, and sets the head MRI image 15 as a normalized head MRI image 55. The normalization unit 46 outputs the normalized head MRI image 55 to the extraction unit 47.

The reference head MRI image 35 is a head MRI image in which a brain having a reference shape, a reference size, and a reference shade (pixel value) appears. The reference head MRI image 35 is, for example, an image generated by averaging head MRI images 15 of a plurality of healthy persons, or an image generated by computer graphics. The reference head MRI image 35 is an example of a "reference medical image" according to the technique of the present disclosure.

The extraction unit 47 inputs the normalized head MRI image 55 to the segmentation model 36. The segmentation model 36 is a machine learning model that performs so-called semantic segmentation of assigning a label representing each of anatomical regions of a brain, such as a hippocampus, an amygdala, and a frontal lobe, to each pixel of the brain appearing in the normalized head MRI image 55. The extraction unit 47 extracts images 56 of a plurality of anatomical regions of the brain (hereinafter, referred to as anatomical region images) from the normalized head MRI image 55 based on the labels assigned by the segmentation model 36. The extraction unit 47 outputs an anatomical region image group 57 including the plurality of anatomical region images 56 for each of the plurality of anatomical regions to the feature amount derivation unit 48.

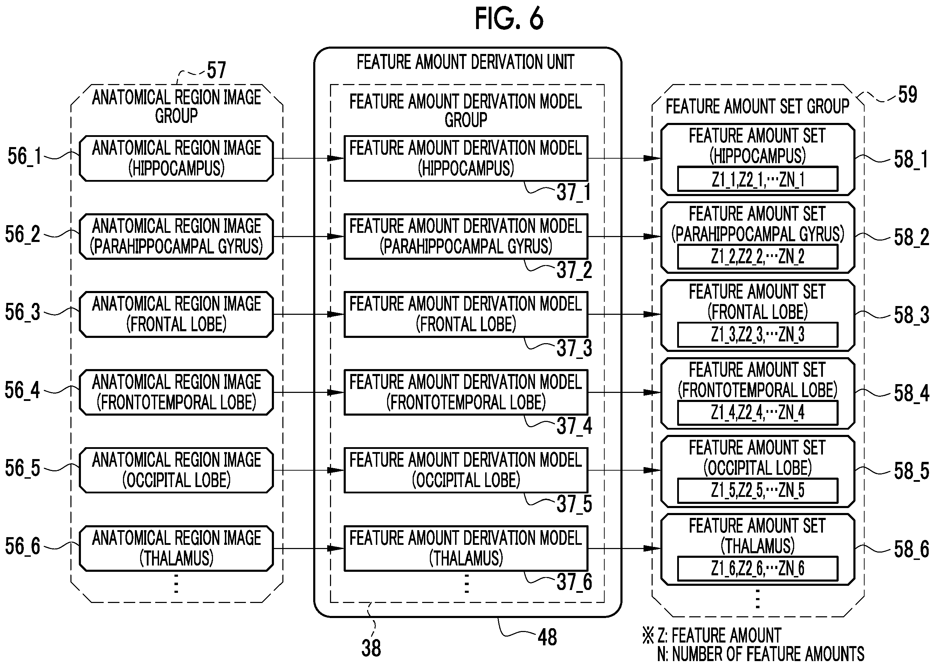

One feature amount derivation model 37 is prepared for each of the plurality of anatomical regions of the brain (refer to Fig. 6). The feature amount derivation unit 48 inputs the anatomical region images 56 to the corresponding feature amount derivation models 37. In addition, a feature amount set 58 including a plurality of types of feature amounts Z (refer to Fig. 6) is output from the feature amount derivation model 37. The feature amount derivation unit 48 outputs a feature amount set group 59 including a plurality of feature amount sets 58 corresponding to the plurality of anatomical regions, to the dementia opinion derivation unit 49.

The dementia opinion derivation unit 49 inputs the feature amount set group 59 to the dementia opinion derivation model 39. In addition, dementia opinion information 60 representing a dementia opinion is output from the dementia opinion derivation model 39. The dementia opinion derivation unit 49 outputs the dementia opinion information 60 to the display control unit 50. The dementia opinion derivation model 39 is an example of a "disease opinion derivation model" according to the technique of the present disclosure.

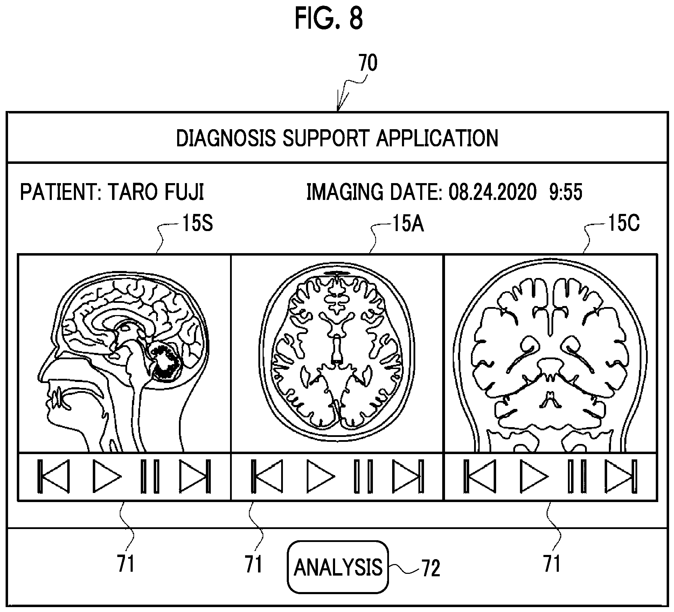

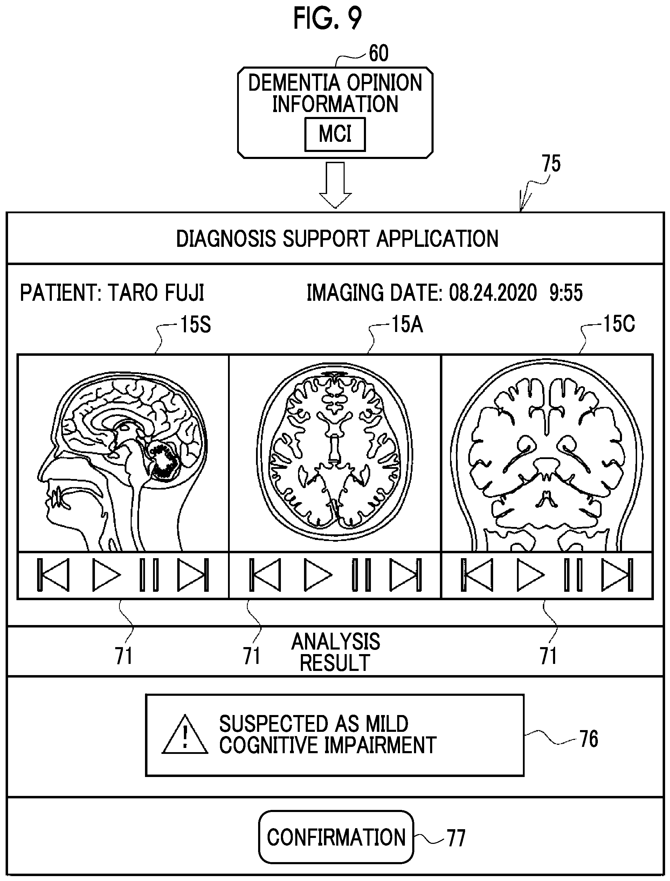

The display control unit 50 controls a display of various screens on the display 17. The various screens include a first display screen 70 (refer to Fig. 8) for instructing analysis by the segmentation model 36, the feature amount derivation model 37, and the dementia opinion derivation model 39, a second display screen 75 (refer to Fig. 9) for displaying the dementia opinion information 60, and the like.

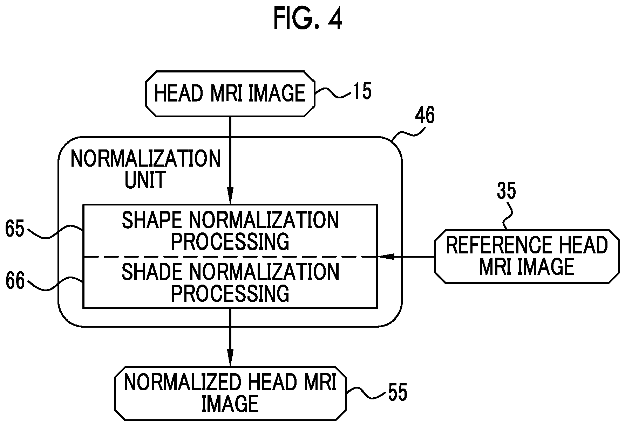

As illustrated in Fig. 4 as an example, the normalization unit 46 performs, as normalization processing, shape normalization processing 65 and shade normalization processing 66 on the head MRI image 15. The shape normalization processing 65 is processing of extracting, for example, landmarks serving as references for registration from the head MRI image 15 and the reference head MRI image 35, and performing parallel displacement, rotation, and/or enlargement/reduction of the head MRI image 15 in accordance with the reference head MRI image 35 such that a correlation between the landmark of the head MRI image 15 and the landmark of the reference head MRI image 35 is maximized. The shade normalization processing 66 is, for example, processing of correcting a shade histogram of the head MRI image 15 in accordance with a shade histogram of the reference head MRI image 35.

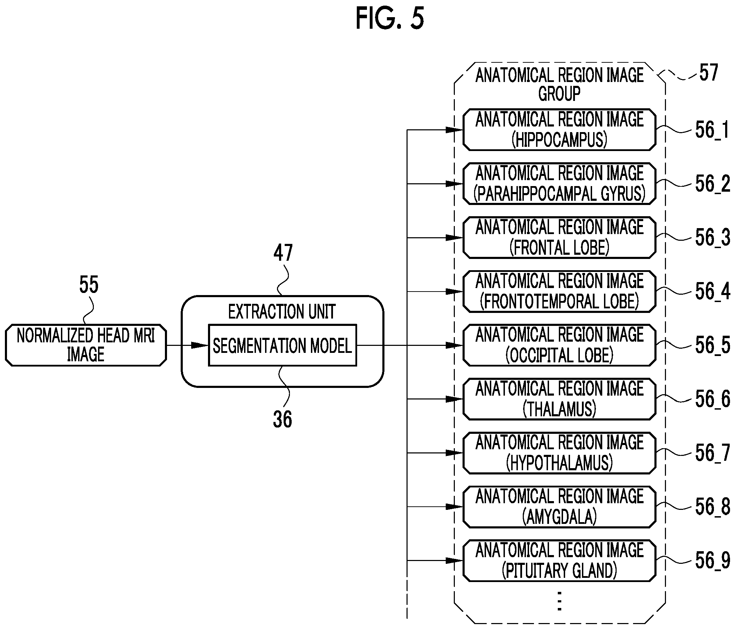

As illustrated in Fig. 5 as an example, the extraction unit 47 extracts, as the anatomical region images 56, the anatomical region image 56_1 of a hippocampus, the anatomical region image 56_2 of a parahippocampal gyrus, the anatomical region image 56_3 of a frontal lobe, the anatomical region image 56_4 of a temporal lobe, the anatomical region image 56_5 of an occipital lobe, the anatomical region image 56_6 of a thalamus, the anatomical region image 56_7 of a hypothalamus, the anatomical region image 56_8 of an amygdala, the anatomical region image 56_9 of a pituitary gland, and the like. In addition to these images, the extraction unit 47 extracts the anatomical region images 56 of the anatomical regions such as mammillary bodies, corpora callosa, fornices, and lateral ventricles. The anatomical regions such as a hippocampus, a frontal lobe, a temporal lobe, and an amygdala come in pairs of a left anatomical region and a right anatomical region. Although not illustrated in the drawings, the anatomical region image 56 of each of the left and right anatomical regions is extracted from the pairs of the left and right anatomical regions. For example, for the hippocampus, the anatomical region image 56_1 of a left hippocampus and the anatomical region image 56_1 of a right hippocampus are extracted. Preferably, the anatomical region includes at least one of a hippocampus or a temporal lobe. More preferably, the anatomical region includes all of a hippocampus and a temporal lobe. The temporal lobe means a front portion of a temporal lobe. For the extraction of the anatomical regions by the extraction unit 47 using the segmentation model 36, for example, a method described in the following literature is used.

<Patrick McClure, etc., Knowing What You Know in Brain Segmentation Using Bayesian Deep Neural Networks, Front. Neuroinform., 17 October 2019.>

As illustrated in Fig. 6 as an example, the feature amount derivation unit 48 inputs the anatomical region image 56_1 of the hippocampus to the feature amount derivation model 37_1 of the hippocampus, and outputs the feature amount set 58_1 of the hippocampus from the feature amount derivation model 37_1 of the hippocampus. The feature amount set 58_1 of the hippocampus includes a plurality of feature amounts Z1_1, Z2_1, ..., ZN_1. N is the number of feature amounts, and is, for example, several tens to hundreds of thousands.

Similarly, the feature amount derivation unit 48 inputs the anatomical region image 56_2 of the parahippocampal gyrus to the feature amount derivation model 37_2 of the parahippocampal gyrus, inputs the anatomical region image 56_3 of the frontal lobe to the feature amount derivation model 37_3 of the frontal lobe, and inputs the anatomical region image 56_4 of the temporal lobe to the feature amount derivation model 37_4 of the temporal lobe. In addition, the feature amount set 58_2 of the parahippocampal gyrus is output from the feature amount derivation model 37_2 of the parahippocampal gyrus, the feature amount set 58_3 of the frontal lobe is output from the feature amount derivation model 37_3 of the frontal lobe, and the feature amount set 58_4 of the temporal lobe is output from the feature amount derivation model 37_4 of the temporal lobe. The feature amount set 58_2 of the parahippocampal gyrus includes a plurality of feature amounts Z1_2, Z2_2, ⋯, ZN_2, the feature amount set 58_3 of the frontal lobe includes a plurality of feature amounts Z1_3, Z2_3, ⋯, ZN_3, and the feature amount set 58_4 of the temporal lobe includes a plurality of feature amounts Z1_4, Z2_4, ⋯, ZN_4.

Further, the feature amount derivation unit 48 inputs the anatomical region image 56_5 of the occipital lobe to the feature amount derivation model 37_5 of the occipital lobe, and inputs the anatomical region image 56_6 of the thalamus to the feature amount derivation model 37_6 of the thalamus. In addition, the feature amount set 58_5 of the occipital lobe is output from the feature amount derivation model 37_5 of the occipital lobe, and the feature amount set 58_6 of the thalamus is output from the feature amount derivation model 37_6 of the thalamus. The feature amount set 58_5 of the occipital lobe includes a plurality of feature amounts Z1_5, Z2_5, ⋯, ZN_5, and the feature amount set 58_6 of the thalamus includes a plurality of feature amounts Z1_6, Z2_6, ⋯, ZN_6. In this way, the plurality of anatomical region images 56 are respectively input to the corresponding feature amount derivation models 37. Thereby, the plurality of feature amount sets 58 for each of the anatomical region images 56 are output from the feature amount derivation models 37. The number of the feature amounts Z may be the same in each anatomical region as in a case of N in the example, or may be different in each anatomical region.

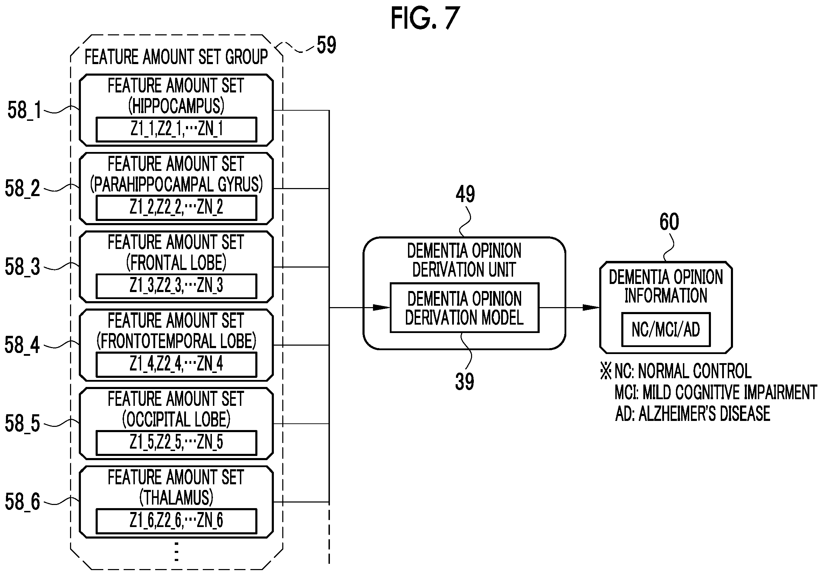

As illustrated in Fig. 7 as an example, the dementia opinion derivation unit 49 inputs the feature amount set group 59 to the dementia opinion derivation model 39. As the dementia opinion information 60, any one of normal control (NC), mild cognitive impairment (MCI), and Alzheimer's disease (AD) is output from the dementia opinion derivation model 39.

Fig. 8 illustrates an example of the first display screen 70 for instructing the analysis by the segmentation model 36, the feature amount derivation model 37, and the dementia opinion derivation model 39. The head MRI images 15 of the patient P for diagnosing dementia are displayed on the first display screen 70. The head MRI images 15 include a head MRI image 15S having a sagittal cross section, a head MRI image 15A having an axial cross section, and a head MRI image 15C having a coronal cross section. A button group 71 for switching the display is provided in a lower portion of each of the head MRI images 15S, 15A, and 15C.

An analysis button 72 is provided on the first display screen 70. The doctor selects the analysis button 72 in a case where he/she wants to perform analysis using the segmentation model 36, the feature amount derivation model 37, and the dementia opinion derivation model 39. In response to the selection, the CPU 22 receives an instruction for analysis by the segmentation model 36, the feature amount derivation model 37, and the dementia opinion derivation model 39.

Fig. 9 illustrates an example of a second display screen 75 for displaying dementia opinion information 60 obtained as a result of analysis by the segmentation model 36, the feature amount derivation model 37, and the dementia opinion derivation model 39. On the second display screen 75, a message 76 according to the dementia opinion information 60 is displayed. Fig. 9 illustrates an example in which the dementia opinion information 60 is mild cognitive impairment (MCI) and "suspected as mild cognitive impairment" is displayed as the message 76. In a case where a confirmation button 77 is selected, the display control unit 50 turns off the display of the message 76, and returns the second display screen 75 to the first display screen 70.

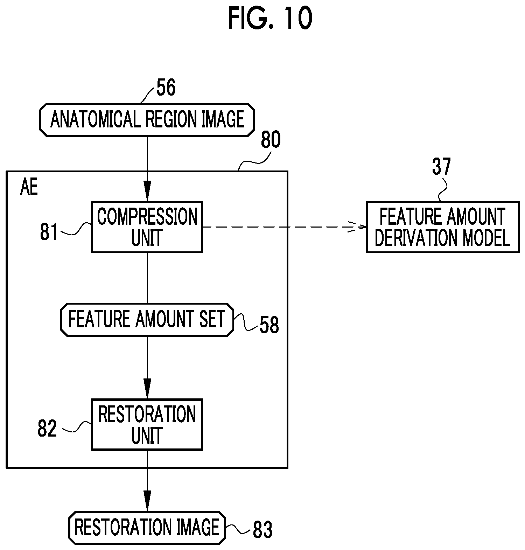

As illustrated in Fig. 10 as an example, a compression unit 81 of an auto-encoder (hereinafter, abbreviated as AE) 80 is used in the feature amount derivation model 37. The AE 80 includes a compression unit 81 and a restoration unit 82. The anatomical region image 56 is input to the compression unit 81. The compression unit 81 converts the anatomical region image 56 into the feature amount set 58. The compression unit 81 transmits the feature amount set 58 to the restoration unit 82. The restoration unit 82 generates a restoration image 83 of the anatomical region image 56 from the feature amount set 58.

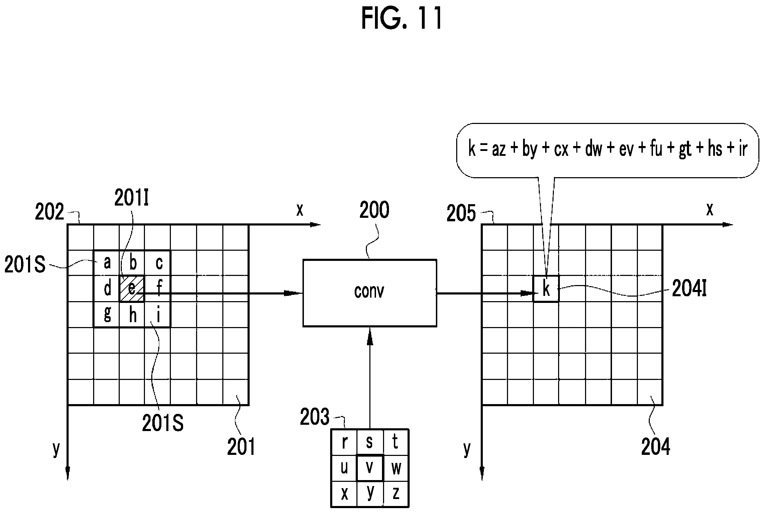

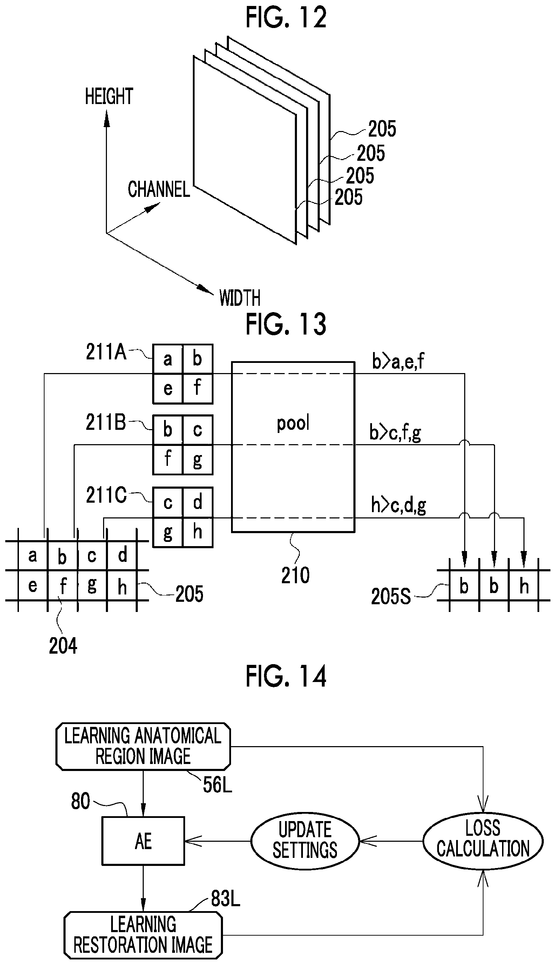

The compression unit 81 converts the anatomical region image 56 into the feature amount set 58 by performing a convolution operation as illustrated in Fig. 11 as an example. Specifically, the compression unit 81 includes a convolutional layer 200 represented by "convolution (abbreviated as conv)". The convolutional layer 200 applies, for example, a $3 \times 3$ filter 203 to the target data 202 including a plurality of elements 201 which are two-dimensionally arranged. In addition, the convolutional layer 200 performs convolution of an element value e of an element of interest 201I, which is one of the elements 201, and element values a, b, c, d, f, g, h, and i of eight elements 201S adjacent to the element of interest 201I. The convolutional layer 200 sequentially performs a convolution operation on each of the elements 201 of the target data 202 while shifting the element of interest 201I by one element, and outputs element values of elements 204 of operation data 205. Thereby, similarly to the target data 202, the operation data 205 including a plurality of elements 204 which are two-dimensionally arranged is obtained. The target data 202 that is first input to the convolutional layer 200 is the anatomical region image 56, and thereafter, reduction operation data 205S (refer to Fig. 13) to be described later is input to the convolutional layer 200 as the target data 202.

In a case where it is assumed that coefficients of the filter 203 are r, s, t, u, v, w, x, y, and z, an element value k of an element 204I of the operation data 205 corresponding to the element of interest 201I is obtained, for example, by calculating the following equation (1), the element value k being a result of the convolution operation on the element of interest 201I.

$$k = az + by + cx + dw + ev + fu + gt + hs + ir \cdots (1)$$

One piece of the operation data 205 is output for one filter 203. In a case where a plurality of types of filters 203 are applied to one piece of the target data 202, the operation data 205 is output for each of the filters 203. That is, as illustrated in Fig. 12 as an example, pieces of the operation data 205 are generated for the number of filters 203 applied to the target data 202. In addition, the operation data 205 includes the plurality of elements 204 which are two-dimensionally arranged, and thus the operation data 205 has a width and a height. The number of pieces of the operation data 205 is called the number of channels. Fig. 12 illustrates four channels of pieces of the operation data 205 that are output by applying the four filters 203 to the target data 202.

As illustrated in Fig. 13 as an example, the compression unit 81 includes a pooling layer 210 represented by "pooling (abbreviated as pool)" in addition to the convolutional layer 200. The pooling layer 210 obtains local statistics of the element values of the elements 204 of the operation data 205, and generates reduction operation data 205 S in which the obtained statistics are used as element values. Here, the pooling layer 210 performs maximum value pooling processing of obtaining, as the local statistic, a maximum value of the element values in a $2 \times 2$ element block 211. By performing the processing while shifting the block 211 by one element in a width direction and a height direction, a size of the reduction operation data 205S is reduced to 1/2 of a size of the original operation data 205. Fig. 13 illustrates a case where the element value b among the element values a, b, e, and f in the block 211A is a maximum value, the element value b among the element values b, c, f, and g in the block 211B is a maximum value, and the element value h among the element values c, d, g, and h in the block 211C is a maximum value. Average value pooling processing of obtaining, as a local statistic, an average value instead of the maximum value may be performed.

The compression unit 81 outputs final operation data 205 by repeating the convolution processing by the convolutional layer 200 and the pooling processing by the pooling layer 210 a plurality of times. The final operation data 205 is, in other words, the feature amount set 58, and the element value of each element 204 of the final operation data 205 is, in other words, the feature amount Z. The feature amount Z obtained in this way represents a shape of the anatomical region and a feature of a texture, such as a degree of atrophy of the hippocampus, a degree of a vascular disorder of a white matter, and the presence or absence of a decrease in blood flow metabolism in the frontal lobe, the temporal lobe, and the occipital lobe. Here, for the sake of simplicity, the description is given that the processing is performed in a two-dimensional manner. On the other hand, the processing is actually performed in a three-dimensional manner.

As illustrated in Fig. 14 as an example, the AE 80 is trained by inputting learning anatomical region images 56L in a learning phase before the compression unit 81 is adapted as the feature amount derivation model 37. The AE 80 outputs learning restoration images 83L in response to the learning anatomical region images 56L. Loss calculation of the AE 80 using a loss function is performed based on the learning anatomical region images 56L and the learning restoration images 83L. In addition, update settings of various coefficients of the AE 80 (such as coefficients of the filters 203) are performed according to a result of the loss calculation, and the AE 80 is updated according to the update settings.

In the learning phase of the AE 80, while exchanging the learning anatomical region images 56L, a series of processing including inputting of the learning anatomical region images 56L to the AE 80, outputting of the learning restoration images 83L from the AE 80, the loss calculation, the update settings, and updating of the AE 80 is repeatedly performed. The repetition of the series of processing is ended in a case where accuracy of restoration from the learning anatomical region images 56L to the learning restoration images 83L reaches a predetermined setting level. The compression unit 81 of the AE 80 of which the restoration accuracy reaches the setting level in this manner is used as the trained feature amount derivation model 37 by being stored in the storage 20.

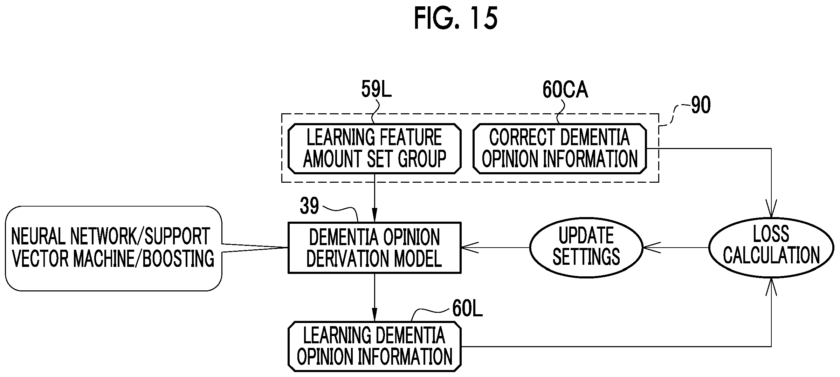

In Fig. 15 illustrating an example of an outline of processing in the learning phase of the dementia opinion derivation model 39, the dementia opinion derivation model 39 is configured by using any one method of a neural network, a support vector machine, and boosting. In the learning phase, the dementia opinion derivation model 39 is trained by inputting learning data 90. The learning data 90 is a set of a learning feature amount set group 59L and correct dementia opinion information 60CA corresponding to the learning feature amount set group 59L. The learning feature amount set group 59L is obtained by inputting the anatomical region image 56 of a certain head MRI image 15 to the feature amount derivation model 37. The correct dementia opinion information 60CA is a result obtained by actually diagnosing, by the doctor, the dementia opinion on the head MRI image 15 from which the learning feature amount set group 59L is obtained.

In the learning phase, the learning feature amount set group 59L is input to the dementia opinion derivation model 39. The dementia opinion derivation model 39 outputs learning dementia opinion information 60L in response to the learning feature amount set group 59L. A loss calculation of the dementia opinion derivation model 39 using a loss function is performed based on the learning dementia opinion information 60L and the correct dementia opinion information 60CA. In addition, update settings of various coefficients of the dementia opinion derivation model 39 are performed according to a result of the loss calculation, and the dementia opinion derivation model 39 is updated according to the update settings.

In the learning phase of the dementia opinion derivation model 39, while exchanging the learning data 90, a series of processing including inputting of the learning feature amount set group 59L to the dementia opinion derivation model 39, outputting of the learning dementia opinion information 60L from the dementia opinion derivation model 39, the loss calculation, the update settings, and updating of the dementia opinion derivation model 39 is repeatedly performed. The repetition of the series of pieces of processing is ended in a case where prediction accuracy of the learning dementia opinion information 60L with respect to the correct dementia opinion information 60CA reaches a predetermined setting level. The dementia opinion derivation model 39 of which the prediction accuracy reaches the setting level in this way is stored in the storage 20, and is used as a trained dementia opinion derivation model in the dementia opinion derivation unit 49.

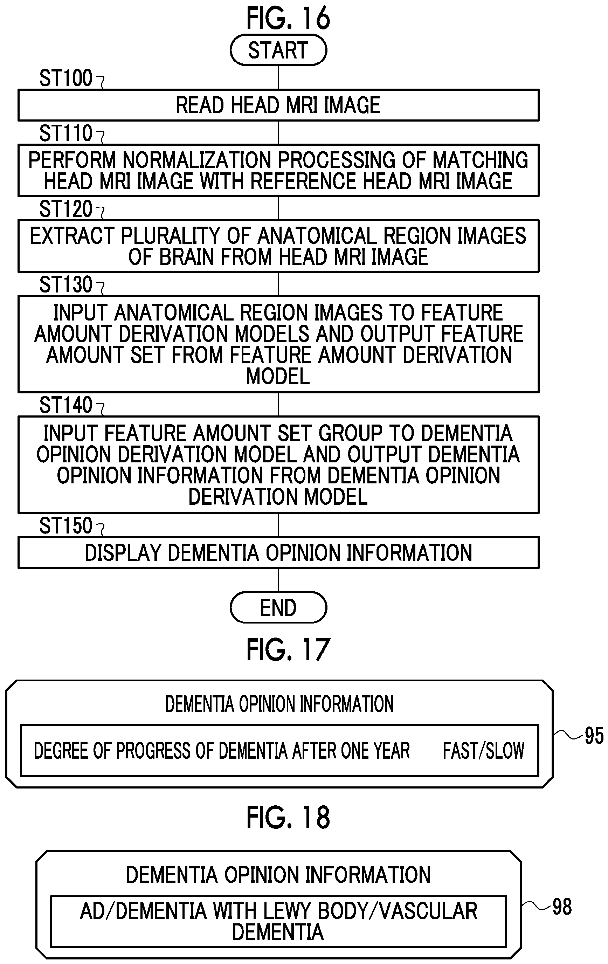

Next, an operation according to the configuration will be described with reference to a flowchart illustrated in Fig. 16. First, in a case where the operation program 30 is started in the diagnosis support device 12, as illustrated in Fig. 3, the CPU 22 of the diagnosis support device 12 functions as the RW control unit 45, the normalization unit 46, the extraction unit 47, the feature amount derivation unit 48, the dementia opinion derivation unit 49, and the display control unit 50.

In a case where the analysis button 72 is selected on the first display screen 70 illustrated in Fig. 8, the RW control unit 45 reads the corresponding head MRI image 15 and the reference head MRI image 35 from the storage 20 (step ST100). The head MRI image 15 and the reference head MRI image 35 are output from the RW control unit 45 to the normalization unit 46.

As illustrated in Fig. 4, the normalization unit 46 performs normalization processing (shape normalization processing 65 and shade normalization processing 66) of matching the head MRI image 15 with the reference head MRI image 35 (step ST110). Thereby, the head MRI image 15 is set as a normalized head MRI image 55. The normalized head MRI image 55 is output from the normalization unit 46 to the extraction unit 47.

As illustrated in Fig. 5, the extraction unit 47 extracts a plurality of anatomical region images 56 from the normalized head MRI image 55 using the segmentation model 36 (step ST120). The anatomical region image group 57 including the plurality of anatomical region images 56 is output from the extraction unit 47 to the feature amount derivation unit 48.

As illustrated in Fig. 6, the feature amount derivation unit 48 inputs the anatomical region images 56 to the corresponding feature amount derivation models 37. Thereby, the feature amount set 58 is output from the feature amount derivation model 37 (step ST130). The feature amount set group 59 including the plurality of feature amount sets 58 is output from the feature amount derivation unit 48 to the dementia opinion derivation unit 49.

As illustrated in Fig. 7, the dementia opinion derivation unit 49 inputs the feature amount set group 59 to the dementia opinion derivation model 39. Thereby, the dementia opinion information 60 is output from the dementia opinion derivation model 39 (step ST140). The dementia opinion information 60 is output from the dementia opinion derivation unit 49 to the display control unit 50.

Under a control of the display control unit 50, the second display screen 75 illustrated in Fig. 9 is displayed on the display 17 (step ST150). A doctor confirms the dementia opinion information 60 via the message 76 on the second display screen 75.

As described above, the CPU 22 of the diagnosis support device 12 includes the RW control unit 45, the extraction unit 47, the feature amount derivation unit 48, the dementia opinion derivation unit 49, and the display control unit 50. The RW control unit 45 acquires the head MRI image 15 by reading the head MRI image 15 of the patient P for diagnosing dementia from the storage 20. The extraction unit 47 extracts the anatomical region images 56 of the plurality of anatomical regions of the brain from the normalized head MRI image 55. The feature amount derivation unit 48 inputs the plurality of anatomical region images 56 to the plurality of feature amount derivation models 37 prepared for each of the plurality of anatomical regions, and outputs the plurality of feature amount sets 58 for each of the plurality of anatomical regions from the feature amount derivation models 37. The dementia opinion derivation unit 49 inputs the feature amount set group 59 including the plurality of feature amount sets 58 to the dementia opinion derivation model 39, and outputs the dementia opinion information 60 from the dementia opinion derivation model 39. The display control unit 50 presents the dementia opinion information 60 to the doctor on the second display screen 75.

The number of feature amounts Z is very large, for example, several tens to hundreds of thousands. For this reason, the feature amount Z does not represent a limited feature of the anatomical region as in the Z value described in JP6483890B, but represents a comprehensive feature of the anatomical region. In addition, the feature amount Z is not a single value which is statistically obtained as in the Z value described in JP6483890B, but is obtained by inputting the anatomical region image 56 to the feature amount derivation model 37. Therefore, according to the method of the present disclosure for deriving the dementia opinion information 60 based on the feature amounts Z (the feature amount set group 59 including the plurality of feature amount sets 58), it is possible to improve the prediction accuracy of the dementia opinion as compared with the method described in JP6483890B. Thereby, it is possible to obtain a more accurate dementia opinion.

In dementia, as compared with other diseases such as cancer, specific lesions that can be recognized with the naked eye are less likely to appear in the image. In addition, dementia has an effect on the entire brain, and is not local. Because of this background, in the related art, it is difficult to obtain an accurate dementia opinion from a medical image such as a head MRI image 15 by using a machine learning model. On the other hand, according to the technique of the present disclosure, the brain is subdivided into the plurality of anatomical regions, feature amounts are derived for each of the plurality of anatomical regions, and the derived feature amounts are input to one dementia opinion derivation model 39. Therefore, it is possible to achieve the object for obtaining a more accurate dementia opinion, as compared with the technique in the related art in which it is difficult to obtain an accurate dementia opinion.

As illustrated in Fig. 10, the feature amount derivation model 37 is obtained by adapting the compression unit 81 of the AE 80. The AE 80 is one of neural network models which are frequently used in the field of machine learning, and is generally very well known. Therefore, the compression unit 81 of the AE 80 can be relatively easily adapted as the feature amount derivation model 37.

As illustrated in Fig. 15, the dementia opinion derivation model 39 is configured by any method of a neural network, a support vector machine, and boosting. Any method of a neural network, a support vector machine, and boosting is generally very well known. Therefore, the dementia opinion derivation model 39 can be relatively easily configured.

As illustrated in Fig. 4, the normalization unit 46 performs normalization processing of matching the head MRI image 15 with the reference head MRI image 35, prior to extraction of the anatomical regions. Therefore, after an individual difference of the patient P and an apparatus difference of the MRI apparatus 10 are substantially eliminated, subsequent processing can be performed. Thereby, it is possible to improve reliability of the dementia opinion information 60.

The dementia has become a social problem with the advent of an aging society in recent years. Therefore, it can be said that the present embodiment of outputting the dementia opinion information 60 in which a brain is set as an organ and dementia is set as a disease is a form that matches the current social problem.

The hippocampus and the temporal lobe are anatomical regions that are particularly highly correlated with dementia such as Alzheimer's disease. Therefore, as in the present example, in a case where the plurality of anatomical regions include at least one of the hippocampus or the temporal lobe, it is possible to obtain a more accurate dementia opinion.

The presentation form of the dementia opinion information 60 is not limited to the second display screen 75. The dementia opinion information 60 may be printed out on a paper medium, or the dementia opinion information 60 may be transmitted to a mobile terminal of the doctor as an attachment file of an e-mail.

The dementia opinion information 60 is not limited to the content illustrated in Fig. 7 (normal control/mild cognitive impairment/Alzheimer's disease). For example, as in the dementia opinion information 95 illustrated in Fig. 17, the dementia opinion information may indicate whether a degree of progression of dementia of the patient P one year later is fast or slow. Alternatively, as in the dementia opinion information 98 illustrated in Fig. 18, the dementia opinion information may be a type of dementia, such as Alzheimer's disease, dementia with Lewy body, or vascular dementia.

[Second Embodiment]

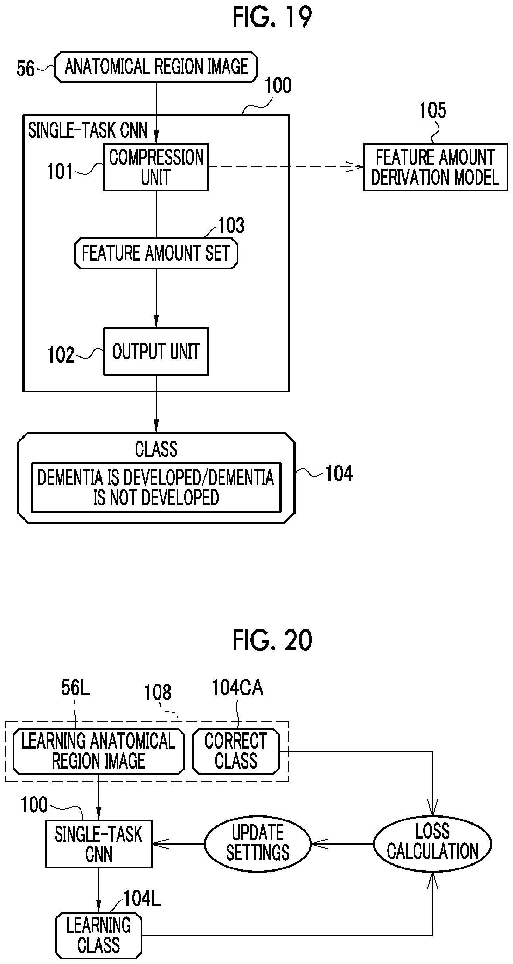

In the second embodiment illustrated in Fig. 19 and Fig. 20, instead of the compression unit 81 of the AE 80, a compression unit 101 of a single-task convolutional neural network for class determination (hereinafter, abbreviated as a single-task CNN) 100 is used as a feature amount derivation model 105.

As illustrated in Fig. 19 as an example, the single-task CNN 100 includes a compression unit 101 and an output unit 102. The anatomical region image 56 is input to the compression unit 101. Similar to the compression unit 81, the compression unit 101 converts the anatomical region image 56 into a feature amount set 103. The compression unit 101 transmits the feature amount set 103 to the output unit 102. The output unit 102 outputs one class 104 based on the feature amount set 103. In Fig. 19, the output unit 102 outputs, as the class 104, a determination result indicating whether dementia is developed or not developed. The compression unit 101 of the single-task CNN 100 is used as the feature amount derivation model 105.

As illustrated in Fig. 20 as an example, the single-task CNN 100 is trained by inputting learning data 108 in a learning phase before the compression unit 101 is adapted as the feature amount derivation model 105. The learning data 108 is a set of the learning anatomical region image 56L and a correct class 104CA corresponding to the learning anatomical region image 56L. The correct class 104CA is a result obtained by actually determining, by the doctor, whether or not dementia is developed on the head MRI image 15 from which the learning anatomical region image 56L is obtained.

In the learning phase, the learning anatomical region image 56L is input to the single-task CNN 100. The single-task CNN 100 outputs a learning class 104L in response to the learning anatomical region image 56L. The loss calculation of the single-task CNN 100 is performed based on the learning class 104L and the correct class 104CA. In addition, update settings of various coefficients of the single-task CNN 100 are performed according to a result of the loss calculation, and the single-task CNN 100 is updated according to the update settings.

In the learning phase of the single-task CNN 100, while exchanging the learning data 108, a series of processing including inputting of the learning anatomical region image 56L to the single-task CNN 100, outputting of the learning class 104L from the single-task CNN 100, the loss calculation, the update settings, and updating of the single-task CNN 100 is repeatedly performed. The repetition of the series of processing is ended in a case where prediction accuracy of the learning class 104L with respect to the correct class 104CA reaches a predetermined setting level. The compression unit 101 of the single-task CNN 100 of which the prediction accuracy reaches the setting level is stored in the storage 20 as the trained feature amount derivation model 105, and is used in the feature amount derivation unit 48.

As described above, in the second embodiment, the compression unit 101 of the single-task CNN 100 is used as the feature amount derivation model 105. The single-task CNN 100 is also one of neural network models which are frequently used in the field of machine learning, and is generally very well known. Therefore, the compression unit 101 of the single-task CNN 100 can be relatively easily adapted as the feature amount derivation model 105.

The class 104 may include, for example, content indicating that the patient P is younger than 75 years old or content indicating that the patient P is 75 years old or older, or may include an age group of the patient P such as 60's and 70's.

[Third Embodiment]

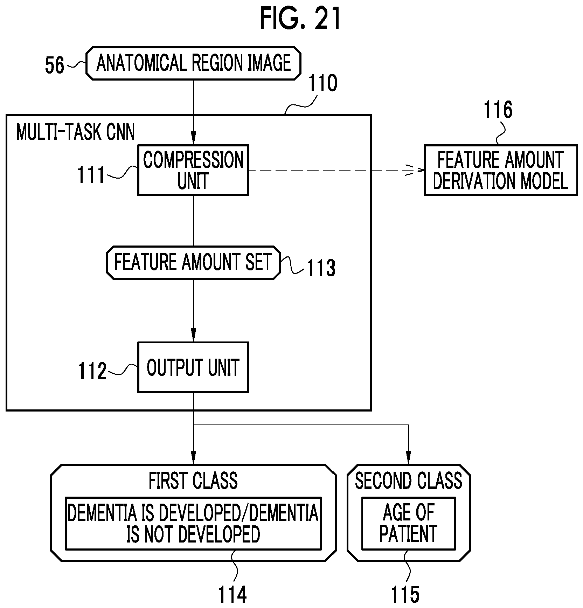

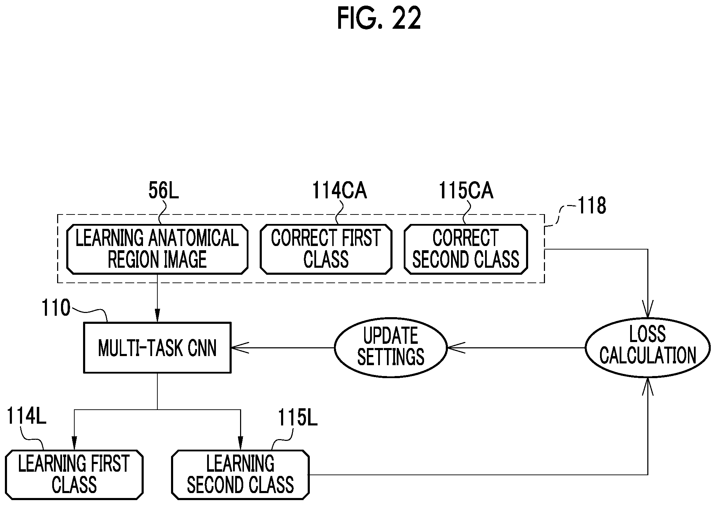

In the third embodiment illustrated in Fig. 21 and Fig. 22, instead of the compression unit 81 of the AE 80 and the compression unit 101 of the single-task CNN 100, a compression unit 111 of a multi-task class determination CNN (hereinafter, abbreviated as a multi-task CNN) 110 is used as a feature amount derivation model 116.

As illustrated in Fig. 21 as an example, the multi-task CNN 110 includes a compression unit 111 and an output unit 112. The anatomical region image 56 is input to the compression unit 111. The compression unit 111 converts the anatomical region image 56 into a feature amount set 113 in the same manner as the compression unit 81 and the compression unit 101. The compression unit 111 transmits the feature amount set 113 to the output unit 112. The output unit 112 outputs two classes of a first class 114 and a second class 115 based on the feature amount set 113. In Fig. 21, the output unit 112 outputs, as the first class 114, a determination result indicating whether dementia is developed or not developed. Further, in Fig. 21, the output unit 112 outputs, as the second class 115, the age of the patient P. The compression unit 111 of the multi-task CNN 110 is used as a feature amount derivation model 116.

As illustrated in Fig. 22 as an example, the multi-task CNN 110 is trained by inputting learning data 118 in a learning phase before the compression unit 111 is adapted as the feature amount derivation model 116. The learning data 118 is a set of the learning anatomical region image 56L and a correct first class 114CA and a correct second class 115CA corresponding to the learning anatomical region image 56L. The correct first class 114CA is a result obtained by actually determining, by the doctor, whether or not dementia is developed on the head MRI image 15 from which the learning anatomical region image 56L is obtained. In addition, the correct second class 115CA is the actual age of the patient P whose the head MRI image 15 is imaged, the head MRI image 15 being an image from which the learning anatomical region image 56L is obtained.

In the learning phase, the learning anatomical region image 56L is input to the multi-task CNN 110. The multi-task CNN 110 outputs a learning first class 114L and a learning second class 115L in response to the learning anatomical region image 56L. The loss calculation of the multi-task CNN 110 is performed based on the learning first class 114L and the learning second class 115L, and the correct first class 114CA and the correct second class 115CA. In addition, update settings of various coefficients of the multi-task CNN 110 are performed according to a result of the loss calculation, and the multi-task CNN 110 is updated according to the update settings.

In the learning phase of the multi-task CNN 110, while exchanging the learning data 118, a series of processing including inputting of the learning anatomical region image 56L to the multi-task CNN 110, outputting of the learning first class 114L and the learning second class 115L from the multi-task CNN 110, the loss calculation, the update settings, and updating of the multi-task CNN 110 is repeatedly performed. The repetition of the series of processing is ended in a case where prediction accuracy of the learning first class 114L and the learning second class 115L with respect to the correct first class 114CA and the correct second class 115CA reaches a predetermined setting level. The compression unit 111 of the multi-task CNN 110 of which the prediction accuracy reaches the setting level is stored in the storage 20 as the trained feature amount derivation model 116, and is used in the feature amount derivation unit 48.

As described above, in the third embodiment, the compression unit 111 of the multi-task CNN 110 is used as the feature amount derivation model 116. The multi-task CNN 110 performs more complicated processing of outputting a plurality of classes (the first class 114 and the second class 115) as compared with the AE 80 and the single-task CNN 100. For this reason, there is a high possibility that the feature amount set 113 output from the compression unit 111 more comprehensively represents a feature of the anatomical region image 56. Therefore, as a result, it is possible to further improve the prediction accuracy of the dementia opinion by the dementia opinion derivation model 39.

The first class 114 may be, for example, a degree of progression of dementia in five levels. In addition, the second class 115 may be a determination result of the age group of the patient P. The multi-task CNN 110 may output three or more classes.

[Fourth Embodiment]

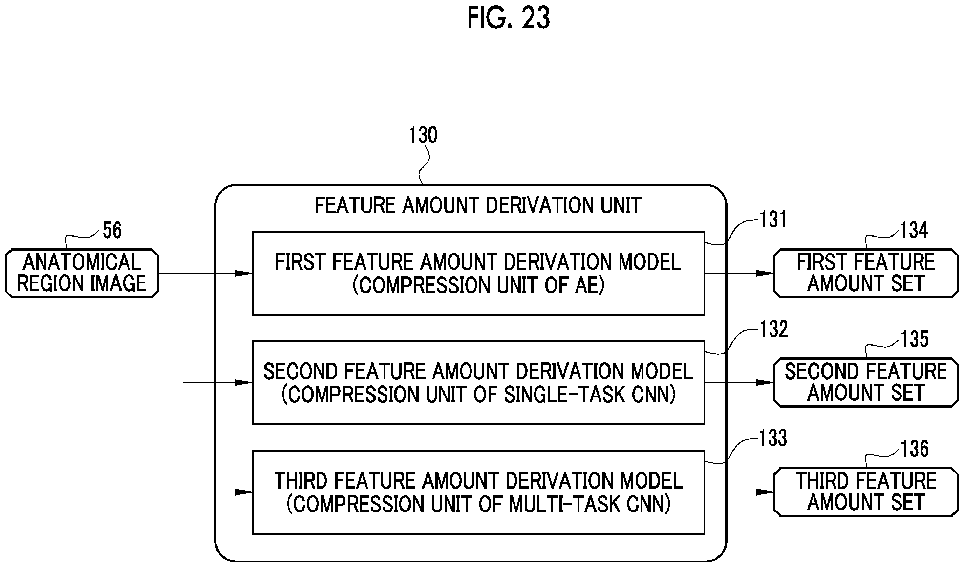

In the fourth embodiment illustrated in Fig. 23, the anatomical region image 56 of one anatomical region is input to a plurality of different feature amount derivation models.

In Fig. 23, the feature amount derivation unit 130 according to the present embodiment inputs the anatomical region image 56 of one anatomical region to a first feature amount derivation model 131, a second feature amount derivation model 132, and a third feature amount derivation model 133. Thereby, the feature amount derivation unit 130 outputs a first feature amount set 134 from the first feature amount derivation model 131, outputs a second feature amount set 135 from the second feature amount derivation model 132, and outputs a third feature amount set 136 from the third feature amount derivation model 133. The first feature amount derivation model 131 is obtained by adapting the compression unit 81 of the AE 80 according to the first embodiment. The second feature amount derivation model 132 is obtained by adapting the compression unit 101 of the single-task CNN 100 according to the second embodiment. The third feature amount derivation model 133 is obtained by adapting the compression unit 111 of the multi-task CNN 110 according to the third embodiment.

As described above, in the fourth embodiment, the feature amount derivation unit 130 inputs the anatomical region image 56 of one anatomical region to the first feature amount derivation model 131, the second feature amount derivation model 132, and the third feature amount derivation model 133. In addition, the first feature amount set 134, the second feature amount set 135, and the third feature amount set 136 are output from each of the models 131 to 133. Therefore, a wide variety of feature amounts Z can be obtained as compared with a case where one kind of feature amount derivation model 37 is used. As a result, it is possible to further improve the prediction accuracy of the dementia opinion by the dementia opinion derivation model 39.

The plurality of different feature amount derivation models may be, for example, a combination of the first feature amount derivation model 131 obtained by adapting the compression unit 81 of the AE 80 and the second feature amount derivation model 132 obtained by adapting the compression unit 101 of the single-task CNN 100. Alternatively, a combination of the second feature amount derivation model 132 obtained by adapting the compression unit 101 of the single-task CNN 100 and the third feature amount derivation model 133 obtained by adapting the compression unit 111 of the multi-task CNN 110 may be used. Further, a combination of the second feature amount derivation model 132, which outputs whether or not dementia is developed as the class 104 and is obtained by adapting the compression unit 101 of the single-task CNN 100, and the second feature amount derivation model 132, which outputs the age group of the patient P as the class 104 and is obtained by adapting the compression unit 101 of the single-task CNN 100, may be used.

[Fifth Embodiment]

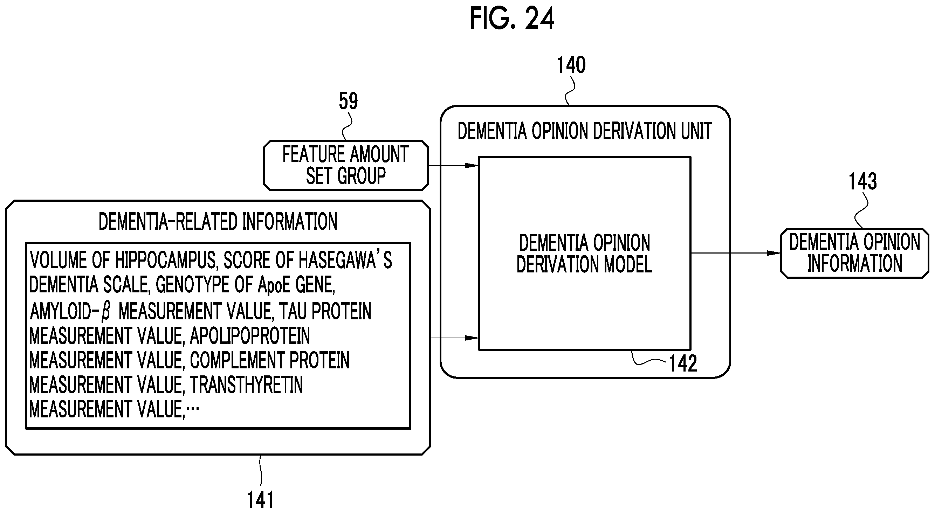

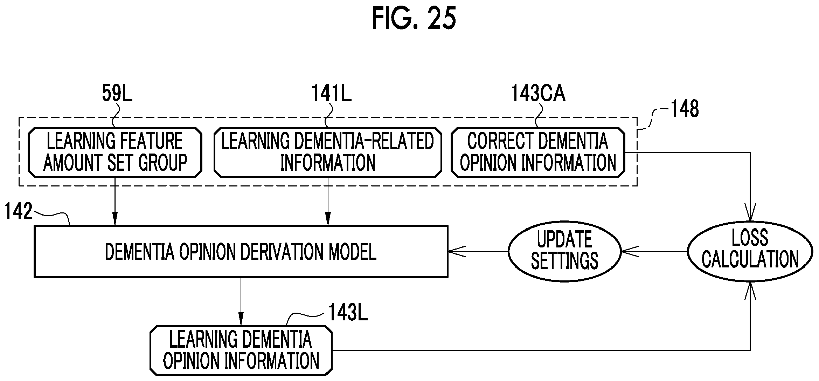

In the fifth embodiment illustrated in Fig. 24 and Fig. 25, dementia-related information 141 related to dementia is input to the dementia opinion derivation model 142 in addition to the plurality of feature amounts Z.

As illustrated in Fig. 24 as an example, the dementia opinion derivation unit 140 according to the present embodiment inputs dementia-related information 141 related to dementia to the dementia opinion derivation model 142 in addition to the feature amount set group 59. In addition, dementia opinion information 143 is output from the dementia opinion derivation model 142. The dementia-related information 141 is an example of "disease-related information" according to the technique of the present disclosure.

The dementia-related information 141 is information on the patient P for diagnosing dementia. The dementia-related information 141 includes, for example, a volume of the hippocampus. In addition, the dementia-related information 141 includes a score of a Hasegawa's dementia scale, a genotype of an ApoE gene, an amyloid-β measurement value, a tau protein measurement value, an apolipoprotein measurement value, a complement protein measurement value, a transthyretin measurement value, and the like. The score of the Hasegawa's dementia scale, the genotype of the ApoE gene, the amyloid-β measurement value, the tau protein measurement value, the apolipoprotein measurement value, the complement protein measurement value, the transthyretin measurement value, and the like are quoted from an electronic chart server that is not illustrated.

The volume of the hippocampus is, for example, the total number of pixels of the anatomical region image 56_1 of the hippocampus. The volume of the hippocampus is an example of a "volume of the anatomical region" according to the technique of the present disclosure. In addition to or instead of the volume of the hippocampus, a volume of another anatomical region such as the amygdala may be included in the dementia-related information 141.

The score of the Hasegawa's dementia scale is an example of a "score of a dementia test" according to the technique of the present disclosure. The dementia-related information 141 may include, in addition to or instead of the score of the Hasegawa's dementia scale, a score of mini-mental state examination (MMSE), a score of a rivermead behavioural memory test (RBMT), clinical dementia rating (CDR), activities of daily living (ADL), and/or Alzheimer's disease assessment scale-cognitive subscale (ADAS-Cog).

The genotype of the ApoE gene is a combination of two types among three types of ApoE genes of ε2, ε3, and ε4 (ε2 and ε3, ε3 and ε4, and the like). A risk of development of the Alzheimer's disease having a genotype including one or two ε4 (ε2 and ε4, ε4 and ε4, and the like) is approximately 3 times to 12 times a risk of development of the Alzheimer's disease having a genotype without ε4 (ε2 and ε3, ε3 and ε3, and the like). The genotype of the ApoE gene is converted into a numerical value. For example, a combination of ε2 and ε3 is converted into 1, and a combination of ε3 and ε3 is converted into 2. The numerical value is input to the dementia opinion derivation model 142. The genotype of the ApoE gene is an example of a "test result of a genetic test" according to the technique of the present disclosure.

The amyloid-β measurement value and the tau protein measurement value are an example of a "test result of a spinal fluid test" according to the technique of the present disclosure. In addition, the apolipoprotein measurement value, the complement protein measurement value, and the transthyretin measurement value are an example of a "test result of a blood test" according to the technique of the present disclosure.

Fig. 25 illustrates an example of an outline of processing in a learning phase of the dementia opinion derivation model 142. The dementia opinion derivation model 142 is trained by inputting learning data 148. The learning data 148 is a combination of the learning feature amount set group 59L, the learning dementia-related information 141L, and the correct dementia opinion information 143CA corresponding to the learning feature amount set group 59L and the learning dementia-related information 141L. The learning feature amount set group 59L is obtained by inputting the anatomical region image 56 of a certain head MRI image 15 to the feature amount derivation model 37. The learning dementia-related information 141L is information of the patient P whose the head MRI image 15 is imaged, the head MRI image 15 being an image from which the learning feature amount set group 59L is obtained. The correct dementia opinion information 143CA is a result obtained by actually diagnosing, by the doctor, the dementia opinion on the head MRI image 15 from which the learning feature amount set group 59L is obtained in consideration of the learning dementia-related information 141L.

In the learning phase, the learning feature amount set group 59L and the learning dementia-related information 141L are input to the dementia opinion derivation model 142. The dementia opinion derivation model 142 outputs the learning dementia opinion information 143L in response to the learning feature amount set group 59L and the learning dementia-related information 141L. A loss calculation of the dementia opinion derivation model 142 using a loss function is performed based on the learning dementia opinion information 143L and the correct dementia opinion information 143CA. In addition, update settings of various coefficients of the dementia opinion derivation model 142 are performed according to a result of the loss calculation, and the dementia opinion derivation model 142 is updated according to the update settings.

In the learning phase of the dementia opinion derivation model 142, while exchanging the learning data 148, a series of processing including inputting of the learning feature amount set group 59L and the learning dementia-related information 141L to the dementia opinion derivation model 142, outputting of the learning dementia opinion information 143L from the dementia opinion derivation model 142, the loss calculation, the update settings, and updating of the dementia opinion derivation model 142 is repeatedly performed. The repetition of the series of pieces of processing is ended in a case where prediction accuracy of the learning dementia opinion information 143L with respect to the correct dementia opinion information 143CA reaches a predetermined setting level. The dementia opinion derivation model 142 of which the prediction accuracy reaches the setting level in this way is stored in the storage 20, and is used as a trained dementia opinion derivation model in the dementia opinion derivation unit 140.

As described above, in the fifth embodiment, the dementia-related information 141 is input to the dementia opinion derivation model 142. The dementia-related information 141 includes a volume of a hippocampus, a score of a Hasegawa's dementia scale, a genotype of an ApoE gene, an amyloid-β measurement value, a tau protein measurement value, an apolipoprotein measurement value, a complement protein measurement value, a transthyretin measurement value, and the like. Pieces of powerful information useful for prediction of a dementia opinion such as the dementia-related information 141 are added. Thus, as compared with the case where the dementia opinions are predicted by using only the feature amount set group 59, it is possible to dramatically improve the prediction accuracy of the dementia opinion.

The dementia-related information 141 may include at least one of a volume of the anatomical region, a score of a dementia test, a test result of a genetic test, a test result of a spinal fluid test, or a test result of a blood test. The dementia-related information 141 may include a gender, an age, and a medical history of the patient P, whether or not the patient P has a relative who develops dementia, and the like.

[Sixth Embodiment]

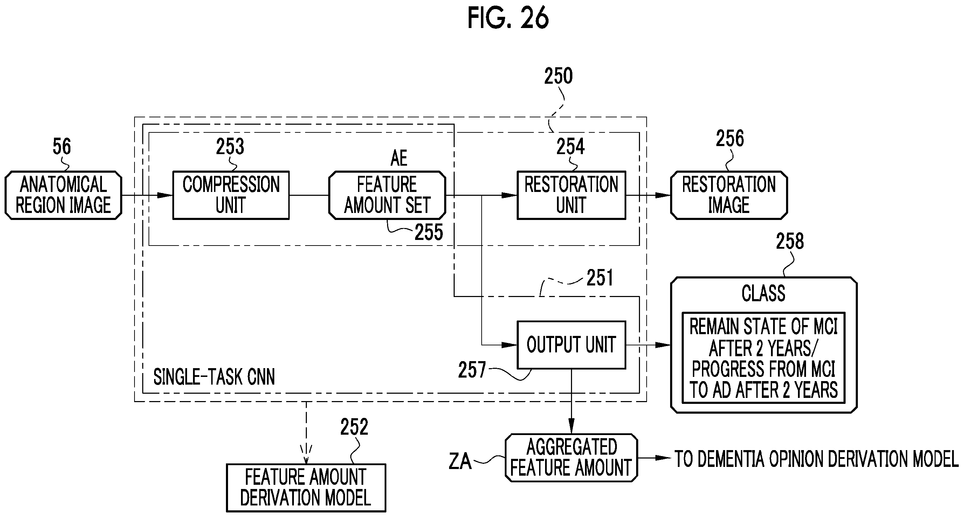

In the sixth embodiment illustrated in Fig. 26 to Fig. 31, a model obtained by combining the AE 250 and the single-task CNN 251 is used as a feature amount derivation model 252.

As illustrated in Fig. 26 as an example, the AE 250 includes a compression unit 253 and a restoration unit 254, similar to the AE 80 according to the first embodiment. The anatomical region image 56 is input to the compression unit 253. The compression unit 253 converts the anatomical region image 56 into the feature amount set 255. The compression unit 253 transmits the feature amount set 255 to the restoration unit 254. The restoration unit 254 generates a restoration image 256 of the anatomical region image 56 from the feature amount set 255.

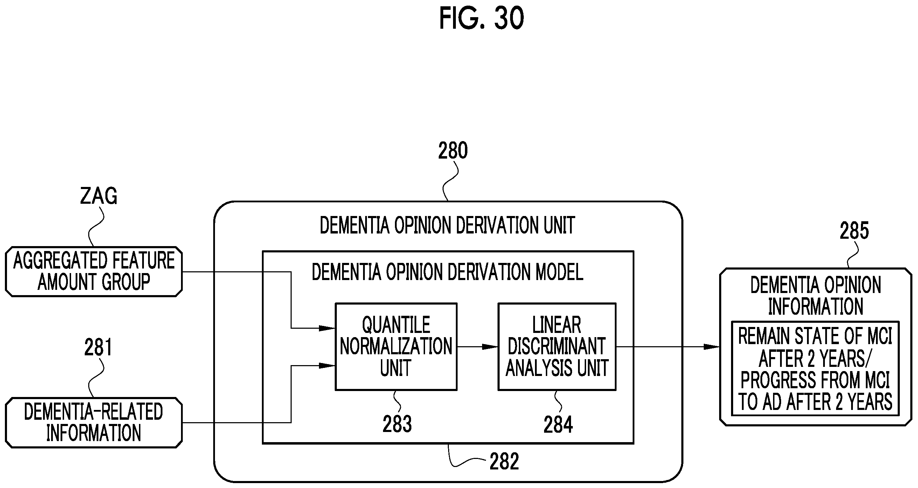

The single-task CNN 251 includes a compression unit 253 and an output unit 257, similar to the single-task CNN 100 according to the second embodiment. That is, the compression unit 253 is shared by the AE 250 and the single-task CNN 251. The compression unit 253 transmits the feature amount set 255 to the output unit 257. The output unit 257 outputs one class 258 based on the feature amount set 255. In Fig. 26, the output unit 257 outputs, as the class 258, a determination result indicating that the patient P with mild cognitive impairment remains a state of mild cognitive impairment after 2 years or progresses to Alzheimer's disease after 2 years. In addition, the output unit 257 outputs aggregated feature amounts ZA obtained by aggregating the plurality of feature amounts Z included in the feature amount set 255. The aggregated feature amounts ZA are output for each of the anatomical regions. In the present embodiment, the aggregated feature amounts ZA are input to the dementia opinion derivation model 282 (refer to Fig. 30) instead of the feature amount set 255.

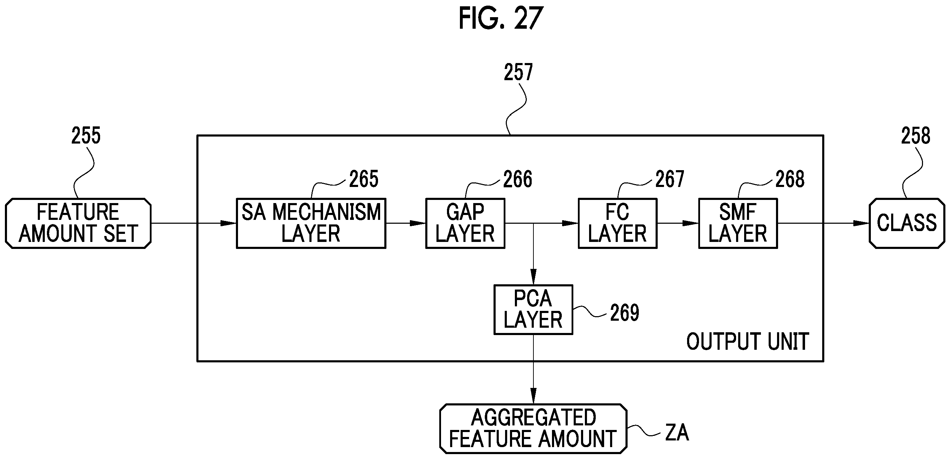

As illustrated in Fig. 27 as an example, the output unit 257 includes a self-attention (hereinafter, abbreviated as SA) mechanism layer 265, a global average pooling (hereinafter, abbreviated as GAP) layer 266, a fully connected (hereinafter, abbreviated as FC) layer 267, a softmax function (hereinafter, abbreviated as SMF) layer 268, and a principal component analysis (hereinafter, abbreviated as PCA) layer 269.

The SA mechanism layer 265 performs convolution processing illustrated in Fig. 11 on the feature amount set 255 while changing the coefficients of the filter 203 according to the element value of the element of interest 201I.

Hereinafter, the convolution processing performed by the SA mechanism layer 265 is referred to as SA convolution processing. The SA mechanism layer 265 outputs the feature amount set 255 after the SA convolution processing to the GAP layer 266.

The GAP layer 266 performs global average pooling processing on the feature amount set 255 after the SA convolution processing. The global average pooling processing is processing of obtaining average values of the feature amounts Z for each channel (refer to Fig. 12) of the feature amount set 255. For example, in a case where the number of channels of the feature amount set 255 is 512, average values of 512 feature amounts Z are obtained by the global average pooling processing. The GAP layer 266 outputs the obtained average values of the feature amounts Z to the FC layer 267 and the PCA layer 269.

The FC layer 267 converts the average values of the feature amounts Z into variables handled by the SMF of the SMF layer 268. The FC layer 267 includes an input layer including units corresponding to the number of the average values of the feature amounts Z (that is, the number of channels of the feature amount set 255) and an output layer including units corresponding to the number of variables handled by the SMF. Each unit of the input layer and each unit of the output layer are fully coupled to each other, and weights are set for each unit. The average values of the feature amounts Z are input to each unit of the input layer. The product sum of the average value of the feature amounts Z and the weight which is set for each unit is an output value of each unit of the output layer. The output value is a variable handled by the SMF. The FC layer 267 outputs the variable handled by the SMF to the SMF layer 268. The SMF layer 268 outputs the class 258 by applying the variable to the SMF.

The PCA layer 269 performs PCA on the average values of the feature amounts Z, and aggregates the average values of the plurality of feature amounts Z into aggregated feature amounts ZA of which the number is smaller than the number of the average values. For example, the PCA layer 269 aggregates the average values of 512 feature amounts Z into one aggregated feature amount ZA.

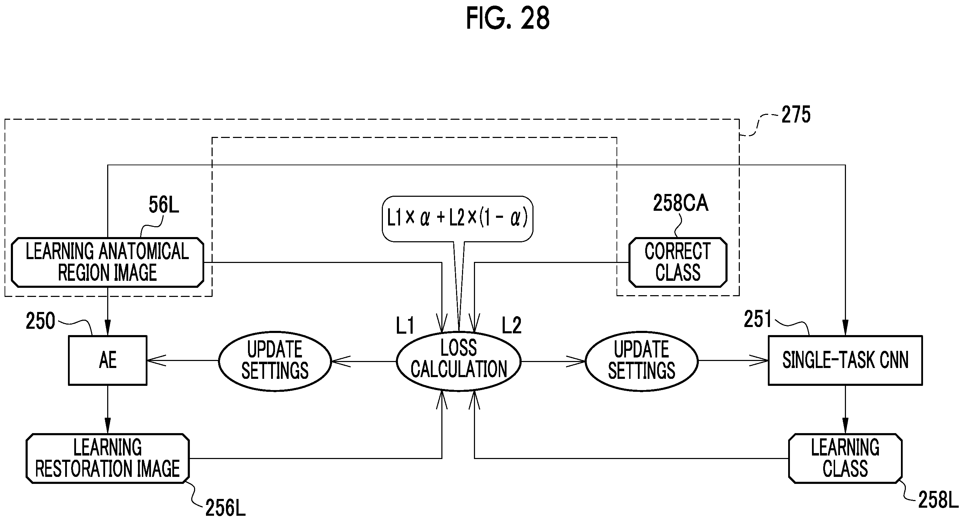

As illustrated in Fig. 28 as an example, the AE 250 is trained by inputting learning anatomical region images 56L in a learning phase. The AE 250 outputs learning restoration images 256L in response to the learning anatomical region images 56L. Loss calculation of the AE 250 using a loss function is performed based on the learning anatomical region images 56L and the learning restoration images 256L. In addition, update settings of various coefficients of the AE 250 are performed according to a result of the loss calculation (hereinafter, referred to as a loss L1), and the AE 250 is updated according to the update settings.

In the learning phase of the AE 250, while exchanging the learning anatomical region images 56L, a series of processing including inputting of the learning anatomical region images 56L to the AE 250, outputting of the learning restoration images 256L from the AE 250, the loss calculation, the update settings, and updating of the AE 250 is repeatedly performed.

The single-task CNN 251 is trained by inputting learning data 275 in a learning phase. The learning data 275 is a set of the learning anatomical region image 56L and a correct class 258CA corresponding to the learning anatomical region image 56L. The correct class 258CA indicates that the patient P whose the head MRI image 15 is imaged and who has mild cognitive impairment remains a state of mild cognitive impairment after 2 years or progresses to Alzheimer's disease after 2 years, the head MRI image 15 being an image from which the learning anatomical region image 56L is obtained.

In the learning phase, the learning anatomical region image 56L is input to the single-task CNN 251. The single-task CNN 251 outputs a learning class 258L in response to the learning anatomical region image 56L. The loss calculation of the single-task CNN 251 using a cross-entropy function or the like is performed based on the learning class 258L and the correct class 258CA. In addition, update settings of various coefficients of the single-task CNN 251 are performed according to a result of the loss calculation (hereinafter, referred to as a loss L2), and the single-task CNN 251 is updated according to the update settings.

In the learning phase of the single-task CNN 251, while exchanging the learning data 275, a series of processing including inputting of the learning anatomical region image 56L to the single-task CNN 251, outputting of the learning class 258L from the single-task CNN 251, the loss calculation, the update settings, and updating of the single-task CNN 251 is repeatedly performed.

The update setting of the AE 250 and the update setting of the single-task CNN 251 are performed based on a total loss L represented by the following equation (2). $\alpha$ is a weight.

$$L = L1 \times \alpha + L2 \times (1-\alpha) \cdots (2)$$

That is, the total loss L is a weighted sum of the loss L1 of the AE 250 and the loss L2 of the single-task CNN 251.

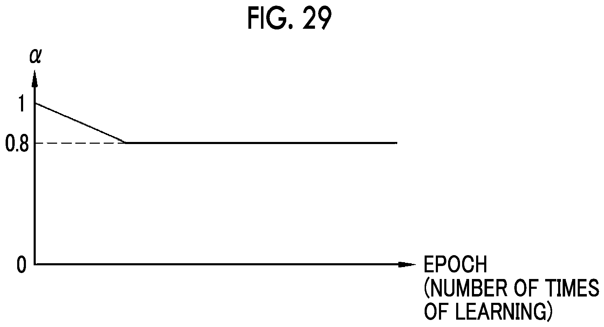

As illustrated in Fig. 29 as an example, the weight $\alpha$ is set to 1 in an initial stage of the learning phase. Assuming that the weight $\alpha$ is 1, the total loss L is represented by L = L1. Therefore, in this case, only the learning of the AE 250 is performed, and the learning of the single-task CNN 251 is not performed.

The weight $\alpha$ is gradually decreased from 1 as the learning is progressed, and is eventually set as a fixed value (0.8 in Fig. 29). In this case, the learning of the AE 250 and the learning of the single-task CNN 251 are both performed with intensity corresponding to the weight $\alpha$. As described above, the weight given to the loss L1 is larger than the weight given to the loss L2. Further, the weight given to the loss L1 is gradually decreased from a maximum value of 1, and the weight given to the loss L2 is gradually increased from a minimum value of 0. Both the weight given to the loss L1 and the weight given to the loss L2 are set as fixed values.

The learning of the AE 250 and the single-task CNN 251 is ended in a case where accuracy of restoration from the learning anatomical region image 56L to the learning restoration image 256L by the AE 250 reaches a predetermined setting level and where prediction accuracy of the learning class 258L with respect to the correct class 258CA by the single-task CNN 251 reaches a predetermined setting level. The AE 250 of which the restoration accuracy reaches the setting level in this way and the single-task CNN 251 of which the prediction accuracy reaches the setting level in this way are stored in the storage 20, and are used as the trained feature amount derivation model 252.

As illustrated in Fig. 30 as an example, the dementia opinion derivation unit 280 according to the present embodiment inputs an aggregated feature amount group ZAG and the dementia-related information 281 to the dementia opinion derivation model 282. The aggregated feature amount group ZAG includes a plurality of aggregated feature amounts ZA which are output for each of the anatomical regions. Similar to the dementia-related information 141 according to the fifth embodiment, the dementia-related information 281 includes a gender and an age of the patient P for diagnosing dementia, a volume of the anatomical region, a score of a dementia test, a test result of a genetic test, a test result of a spinal fluid test, a test result of a blood test, and the like.

The dementia opinion derivation model 282 includes a quantile normalization unit 283 and a linear discriminant analysis unit 284. The aggregated feature amount group ZAG and the dementia-related information 281 are input to the quantile normalization unit 283. The quantile normalization unit 283 performs quantile normalization of converting the plurality of aggregated feature amounts ZA included in the aggregated feature amount group ZAG and each of parameters of the dementia-related information 281 into data according to a normal distribution, in order to handle the plurality of aggregated feature amounts ZA and the parameters in the same sequence. The linear discriminant analysis unit 284 performs linear discriminant analysis on the aggregated feature amounts ZA and each of the parameters of the dementia-related information 281 after the quantile normalization processing, and outputs dementia opinion information 285 as a result of the linear discriminant analysis. The dementia opinion information 285 indicates that the patient P with mild cognitive impairment remains a state of mild cognitive impairment after 2 years or progresses to Alzheimer's disease after 2 years. The learning of the dementia opinion derivation model 282 is the same as the learning of the dementia opinion derivation model 142 illustrated in Fig. 25, except that the learning feature amount set group 59L is changed to the learning aggregated feature amount group ZAG Thus, illustration and description thereof will be omitted.

As described above, in the sixth embodiment, the single-task CNN 251 that performs a main task such as outputting of the class 258 and the AE 250 that is partially common to the single-task CNN 251 and performs a sub-task such as generation of the restoration image 256 are used as the feature amount derivation model 252, the sub-task being a task having a more general purpose as compared with the main task. In addition, the AE 250 and the single-task CNN 251 are trained at the same time. Therefore, as compared with a case where the AE 250 and the single-task CNN 251 are separate, the feature amount set 255 that is more appropriate and the aggregated feature amounts ZA that are more appropriate can be output. As a result, it is possible to improve the prediction accuracy of the dementia opinion information 285.

In the learning phase, the update setting is performed based on the total loss L, which is a weighted sum of the loss L1 of the AE 250 and the loss L2 of the single-task CNN 251. Therefore, by setting the weight $\alpha$ to an appropriate value, the AE 250 can be intensively trained, the single-task CNN 251 can be intensively trained, or the AE 250 and the single-task CNN 251 can be trained in a well-balanced manner.

The weight given to the loss L1 is larger than the weight given to the loss L2. Therefore, the AE 250 can always be intensively trained. In a case where the AE 250 is always intensively trained, the feature amount set 255 that more represents the shape of the anatomical region and the feature of the texture can be output from the compression unit 253. As a result, the aggregated feature amounts ZA having a higher plausibility can be output from the output unit 257.

Further, the weight given to the loss L1 is gradually decreased from the maximum value, and the weight given to the loss L2 is gradually increased from the minimum value. After the learning is performed a predetermined number of times, both the weight given to the loss L1 and the weight given to the loss L2 are set as fixed values. Thus, the AE 250 can be more intensively trained in an initial stage of the learning. The AE 250 is responsible for a relatively simple sub-task such as generation of the restoration image 256. Therefore, in a case where the AE 250 is more intensively trained in the initial stage of the learning, the feature amount set 255 that more represents the shape of the anatomical region and the feature of the texture can be output from the compression unit 253 in the initial stage of the learning.

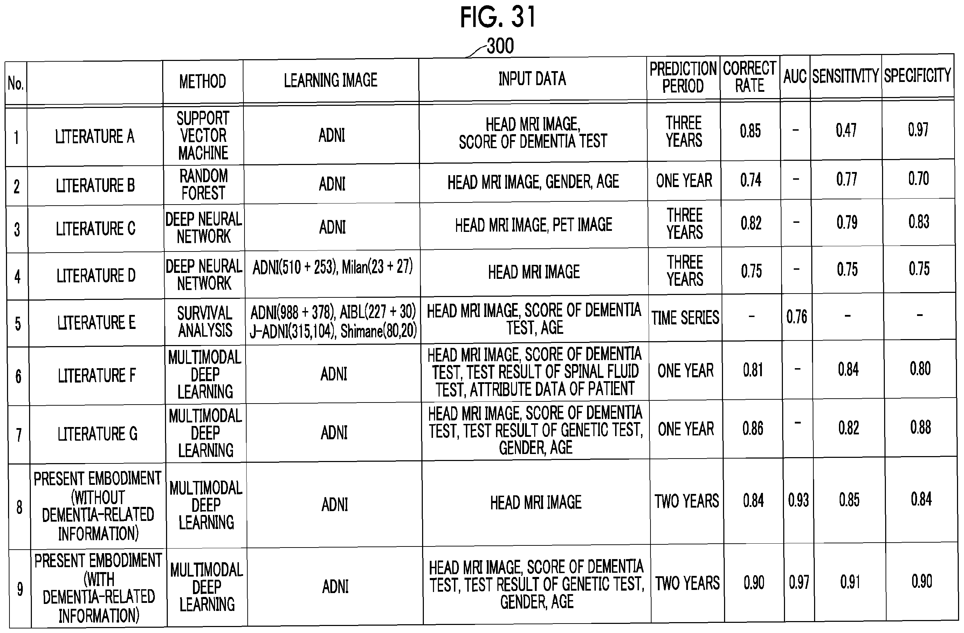

As an example, a table 300 illustrated in Fig. 31 shows performance comparison between Nos. 1 to 7 and Nos. 8 and 9, Nos. 1 to 7 being described in the following literatures A, B, C, D, E, F, and G and being related to a method of predicting progress of dementia, and Nos. 8 and 9 being related to a method of predicting progress of dementia according to the present embodiment. In the method of predicting progress of dementia according to the present embodiment, No. 8 indicates a case where only the aggregated feature amount group ZAG is input to the dementia opinion derivation model 282 and the dementia-related information 281 is not input. On the other hand, No. 9 indicates a case where the aggregated feature amount group ZAG and the dementia-related information 281 are input to the dementia opinion derivation model 282.

Literature A <Tam, A., Dansereau, C., Iturria-Medina, Y, Urchs, S., Orban, P., Sharmarke, H., Breitner, J., & Alzheimer's Disease Neuroimaging Initiative., "A highly predictive signature of cognition and brain atrophy for progression to Alzheimer's dementia.", GigaScience, 8 (5), giz055 (2019).>

Literature B <Ledig, C., Schuh, A., Guerrero, R., Heckemann, R. A., & Rueckert, D., "Structural brain imaging in Alzheimer's disease and mild cognitive impairment: biomarker analysis and shared morphometry database.", Scientific reports, 8 (1), 11258 (2018).>

Literature C <Lu, D., Popuri, K., Ding, G W., Balachandar, R., & Beg, M. F., "Multimodal and multiscale deep neural networks for the early diagnosis of Alzheimer's disease using structural MR and FDG-PET images", Scientific reports, 8 (1), 5697 (2018).>

Literature D <Basaia, S., Agosta, F., Wagner, L., Canu, E., Magnani, G, Santangelo, R., Filippi, M., Automated classification of Alzheimer's disease and mild cognitive impairment using a single MRI and deep neural networks, NeuroImage: Clinical 21, 101645 (2019).>

Literature E <Nakagawa, T., Ishida, M., Naito, J., Nagai, A., Yamaguchi, S., Onoda, K., "Prediction of conversion to Alzheimer's disease using deep survival analysis of MRI images", Brain Communications, Vol. 2 (1) (2020).>

Literature F <Lee, G, Nho, K., Kang, B., Sohn, K. A., & Kim, D., "Predicting Alzheimer's disease progression using multi-modal deep learning approach.", Scientific reports, 9 (1), 1952 (2019).>

Literature G <Goto, T., Wang, C., Li, Y, Tsuboshita, Y, Multi-modal deep learning for predicting progression of Alzheimer's disease using bi-linear shake fusion, Proc. SPIE 11314, Medical Imaging (2020).>

The accuracy of No. 8 and the accuracy of No. 9 are 0.84 and 0.90. In particular, the accuracy of No. 9 is 0.90 and is higher than the accuracy of any one of Nos. 1 to 7. An area under the curve (AUC) of No. 8 and an area under the curve (AUC) of No. 9 are 0.93 and 0.97. These values are larger than a value in No. 5 that is related to a method of predicting progress of dementia and is described in Literature E. Therefore, it can be said that the method of predicting progress of dementia according to the present embodiment can predict progress of dementia with higher accuracy as compared with the methods of predicting progress of dementia in the related art that are described in Literatures A to G

A sensitivity of No. 8 and a sensitivity of No. 9 are 0.85 and 0.91. These values are higher than sensitivities in Nos. 1 to 7. In particular, the sensitivity of No. 9 is 0.91, and is a maximum value among the sensitivities. Therefore, it can be said that the method of predicting progress of dementia according to the present embodiment can predict that the patient P with mild cognitive impairment will progress to Alzheimer's disease after a prediction period without overlooking the progress as compared with the methods of predicting progress of dementia in the related art that are described in Literatures A to G.

A specificity of No. 8 and a specificity of No. 9 are 0.84 and 0.90. These values are smaller than 0.97 in No. 1 related to the method of predicting progress of dementia that is described in Literature A, but are larger than values in other Literatures B, C, D, and F. Therefore, it can be said that the method of predicting progress of dementia according to the present embodiment can more accurately predict that the patient P with mild cognitive impairment remains a state of mild cognitive impairment even after a prediction period as compared with many other methods of predicting progress of dementia in the related art.

In the table 300, ADNI in the items of the learning image is an abbreviation of "Alzheimer's disease Neuroimaging Initiative". AIBL is an abbreviation of "Australian Imaging Biomarkers and Lifestyle Study of Ageing". J-ADNI is an abbreviation of "Japanese Alzheimer's Disease Neuroimaging Intiative". The items indicate a database in which head MRI images 15 and the like of patients P with Alzheimer's disease are accumulated.

Instead of the single-task CNN 251, the multi-task CNN 110 according to the third embodiment may be used.

The learning of the AE 80 illustrated in Fig. 14, the learning of the dementia opinion derivation model 39 illustrated in Fig. 15, the learning of the single-task CNN 100 illustrated in Fig. 20, the learning of the multi-task CNN 110 illustrated in Fig. 22, the learning of the dementia opinion derivation model 142 illustrated in Fig. 25, the learning of the AE 250 and the single-task CNN 251 illustrated in Fig. 28, and the like may be performed by the diagnosis support device 12 or by a device other than the diagnosis support device 12. In addition, the learning may be continuously performed after storing each model in the storage 20 of the diagnosis support device 12.

The PACS server 11 may function as the diagnosis support device 12.

The medical image is not limited to the head MRI image 15 in the example. The medical image may be a positron emission tomography (PET) image, a single photon emission computed tomography (SPECT) image, a computed tomography (CT) image, an endoscopic image, an ultrasound image, or the like.

The organ is not limited to the illustrated brain, and may be a heart, a lung, a liver, or the like. In a case of a lung, right lungs S1 and S2 and left lungs S1 and S2 are extracted as the anatomical regions. In a case of a liver, a right lobe, a left lobe, a gall bladder, and the like are extracted as the anatomical regions. In addition, the disease is not limited to the exemplified dementia, and may be a heart disease, a diffuse lung disease such as interstitial pneumonia, or a dyshepatia such as hepatocirrhosis.

In each of the embodiments, for example, as a hardware structure of the processing unit that executes various processing, such as the RW control unit 45, the normalization unit 46, the extraction unit 47, the feature amount derivation units 48 and 130, the dementia opinion derivation units 49, 140, and 280, and the display control unit 50, the following various processors may be used. The various processors include, as described above, the CPU 22 which is a general-purpose processor that functions as various processing units by executing software (an operation program 30), a programmable logic device (PLD) such as a field programmable gate array (FPGA) which is a processor capable of changing a circuit configuration after manufacture, a dedicated electric circuit such as an application specific integrated circuit (ASIC) which is a processor having a circuit configuration specifically designed to execute specific processing, and the like.

One processing unit may be configured by one of these various processors, or may be configured by a combination of two or more processors having the same type or different types (for example, a combination of a plurality of FPGAs and/or a combination of a CPU and an FPGA). Further, the plurality of processing units may be configured by one processor.

As an example in which the plurality of processing units are configured by one processor, firstly, as represented by a computer such as a client and a server, a form in which one processor is configured by a combination of one or more CPUs and software and the processor functions as the plurality of processing units may be adopted. Secondly, as represented by system on chip (SoC), there is a form in which a processor that realizes the functions of the entire system including a plurality of processing units with one integrated circuit (IC) chip is used. As described above, the various processing units are configured by using one or more various processors as a hardware structure.

Further, as the hardware structure of the various processors, more specifically, an electric circuit (circuitry) in which circuit elements such as semiconductor elements are combined may be used.

The technique of the present disclosure can also appropriately combine the various embodiments and/or the various modification examples. In addition, the technique of the present disclosure is not limited to each embodiment, and various configurations may be adopted without departing from the scope of the present disclosure. Further, the technique of the present disclosure extends to a program and a storage medium for non-temporarily storing the program.

The described contents and the illustrated contents are detailed explanations of a part according to the technique of the present disclosure, and are merely examples of the technique of the present disclosure. For example, the descriptions related to the configuration, the function, the operation, and the effect are descriptions related to examples of a configuration, a function, an operation, and an effect of a part according to the technique of the present disclosure. Therefore, it goes without saying that, in the described contents and illustrated contents, unnecessary parts may be deleted, new components may be added, or replacements may be made without departing from the spirit of the technique of the present disclosure. Further, in order to avoid complications and facilitate understanding of the part according to the technique of the present disclosure, in the described contents and illustrated contents, descriptions of technical knowledge and the like that do not require particular explanations to enable implementation of the technique of the present disclosure are omitted.

In this specification, "A and/or B" is synonymous with "at least one of A or B". That is, "A and/or B" means that only A may be included, that only B may be included, or that a combination of A and B may be included. Further, in this specification, even in a case where three or more matters are expressed by being connected using "and/or", the same concept as "A and/or B" is applied.

All documents, patent applications, and technical standards mentioned in this specification are incorporated herein by reference to the same extent as in a case where each document, each patent application, and each technical standard are specifically and individually described by being incorporated by reference.

**Claims**

1. A diagnosis support device comprising:

   a processor; and
   a memory connected to or built in the processor,
   the processor is configured to:

   acquire a medical image;
   extract a plurality of anatomical regions of an organ from the medical image;
   input images of the plurality of anatomical regions to a plurality of feature amount derivation models prepared for each of the plurality of anatomical regions, and output a plurality of feature amounts for each of the plurality of anatomical regions from the feature amount derivation models;
input the plurality of feature amounts which are output for each of the plurality of anatomical regions to a disease opinion derivation model, and output a disease opinion from the disease opinion derivation model; and
present the opinion.

2. The diagnosis support device according to claim 1,
wherein the feature amount derivation model includes at least one of an auto-encoder, a single-task convolutional neural network for class determination, or a multi-task convolutional neural network for class determination.

3. The diagnosis support device according to claim 1 or 2,
the processor is configured to:
input an image of one anatomical region of the anatomical regions to the plurality of different feature amount derivation models, and output the feature amounts from each of the plurality of feature amount derivation models.

4. The diagnosis support device according to any one of claims 1 to 3,
the processor is configured to:
input disease-related information related to the disease to the disease opinion derivation model in addition to the plurality of feature amounts.

5. The diagnosis support device according to any one of claims 1 to 4,
wherein the disease opinion derivation model is configured by any one method of a neural network, a support vector machine, or boosting.

6. The diagnosis support device according to any one of claims 1 to 5,
the processor is configured to:
perform normalization processing of matching the acquired medical image with a reference medical image prior to extraction of the anatomical regions.

7. The diagnosis support device according to any one of claims 1 to 6,
wherein the organ is a brain and the disease is dementia.

8. The diagnosis support device according to claim 7,
wherein the plurality of anatomical regions include at least one of a hippocampus or a temporal lobe.

9. The diagnosis support device according to claim 7 or 8 citing claim 4,
wherein the disease-related information includes at least one of a volume of the anatomical region, a score of a dementia test, a test result of a genetic test, a test result of a spinal fluid test, or a test result of a blood test.

10. An operation method of a diagnosis support device, the method comprising:

acquiring a medical image;
extracting a plurality of anatomical regions of an organ from the medical image;
inputting images of the plurality of anatomical regions to a plurality of feature amount derivation models prepared for each of the plurality of anatomical regions, and outputting a plurality of feature amounts for each of the plurality of anatomical regions from the feature amount derivation models;
inputting the plurality of feature amounts which are output for each of the plurality of anatomical regions to a disease opinion derivation model, and outputting a disease opinion from the disease opinion derivation model; and
presenting the opinion.

11. An operation program of a diagnosis support device, the program causing a computer to execute a process comprising:

acquiring a medical image;
extracting a plurality of anatomical regions of an organ from the medical image;
inputting images of the plurality of anatomical regions to a plurality of feature amount derivation models prepared for each of the plurality of anatomical regions, and outputting a plurality of feature amounts for each of the plurality of anatomical regions from the feature amount derivation models;

inputting the plurality of feature amounts which are output for each of the plurality of anatomical regions to a disease opinion derivation model, and outputting a disease opinion from the disease opinion derivation model; and

presenting the opinion.

12. A dementia diagnosis support method causing a computer that includes a processor and a memory connected to or built in the processor to execute a process comprising:

acquiring a medical image in which a brain appears;

extracting a plurality of anatomical regions of the brain from the medical image;

inputting images of the plurality of anatomical regions to a plurality of feature amount derivation models prepared for each of the plurality of anatomical regions, and outputting a plurality of feature amounts for each of the plurality of anatomical regions from the feature amount derivation models;

inputting the plurality of feature amounts which are output for each of the plurality of anatomical regions to a dementia opinion derivation model, and outputting a dementia opinion from the dementia opinion derivation model; and

presenting the opinion.

13. A trained dementia opinion derivation model for causing a computer to execute a function of outputting a dementia opinion in response to inputting of a plurality of feature amounts,

wherein the plurality of feature amounts are output from a plurality of feature amount derivation models prepared for each of a plurality of anatomical regions of a brain by inputting images of the plurality of anatomical regions to the plurality of feature amount derivation models, the anatomical regions being extracted from a medical image in which a brain appears.

# FIG. 1

EP 4 223 229 A1

# FIG. 2

| | |
|---|---|
| DISPLAY | ~17 |
| INPUT DEVICE | ~18 |

CPU ~22

MEMORY ~21

STORAGE ~20

COMMUNICATION UNIT ~23

~24

~12

# FIG. 3

## FIG. 4

HEAD MRI IMAGE ~15

46

NORMALIZATION
UNIT

65 ~ SHAPE NORMALIZATION
PROCESSING

35

66 ~ SHADE NORMALIZATION
PROCESSING

REFERENCE HEAD
MRI IMAGE

NORMALIZED HEAD MRI
IMAGE ~55

EP 4 223 229 A1

# FIG. 5

## FIG. 6

FEATURE AMOUNT DERIVATION UNIT

57 — ANATOMICAL REGION IMAGE GROUP

56_1 — ANATOMICAL REGION IMAGE (HIPPOCAMPUS)

56_2 — ANATOMICAL REGION IMAGE (PARAHIPPOCAMPAL GYRUS)

56_3 — ANATOMICAL REGION IMAGE (FRONTAL LOBE)

56_4 — ANATOMICAL REGION IMAGE (FRONTOTEMPORAL LOBE)

56_5 — ANATOMICAL REGION IMAGE (OCCIPITAL LOBE)

56_6 — ANATOMICAL REGION IMAGE (THALAMUS)

FEATURE AMOUNT DERIVATION MODEL GROUP

FEATURE AMOUNT DERIVATION MODEL (HIPPOCAMPUS) — 37_1

FEATURE AMOUNT DERIVATION MODEL (PARAHIPPOCAMPAL GYRUS) — 37_2

FEATURE AMOUNT DERIVATION MODEL (FRONTAL LOBE) — 37_3

FEATURE AMOUNT DERIVATION MODEL (FRONTOTEMPORAL LOBE) — 37_4

FEATURE AMOUNT DERIVATION MODEL (OCCIPITAL LOBE) — 37_5

FEATURE AMOUNT DERIVATION MODEL (THALAMUS) — 37_6

38    48

59 — FEATURE AMOUNT SET GROUP

FEATURE AMOUNT SET (HIPPOCAMPUS) $Z1\_1, Z2\_1, \cdots ZN\_1$ — 58_1

FEATURE AMOUNT SET (PARAHIPPOCAMPAL GYRUS) $Z1\_2, Z2\_2, \cdots ZN\_2$ — 58_2

FEATURE AMOUNT SET (FRONTAL LOBE) $Z1\_3, Z2\_3, \cdots ZN\_3$ — 58_3

FEATURE AMOUNT SET (FRONTOTEMPORAL LOBE) $Z1\_4, Z2\_4, \cdots ZN\_4$ — 58_4

FEATURE AMOUNT SET (OCCIPITAL LOBE) $Z1\_5, Z2\_5, \cdots ZN\_5$ — 58_5

FEATURE AMOUNT SET (THALAMUS) $Z1\_6, Z2\_6, \cdots ZN\_6$ — 58_6

※ Z: FEATURE AMOUNT
N: NUMBER OF FEATURE AMOUNTS

FIG. 7

**FEATURE AMOUNT SET GROUP** — 59

58_1 — FEATURE AMOUNT SET (HIPPOCAMPUS)
$Z1\_1, Z2\_1, \cdots ZN\_1$

58_2 — FEATURE AMOUNT SET (PARAHIPPOCAMPAL GYRUS)
$Z1\_2, Z2\_2, \cdots ZN\_2$

58_3 — FEATURE AMOUNT SET (FRONTAL LOBE)
$Z1\_3, Z2\_3, \cdots ZN\_3$

58_4 — FEATURE AMOUNT SET (FRONTOTEMPORAL LOBE)
$Z1\_4, Z2\_4, \cdots ZN\_4$

58_5 — FEATURE AMOUNT SET (OCCIPITAL LOBE)
$Z1\_5, Z2\_5, \cdots ZN\_5$

58_6 — FEATURE AMOUNT SET (THALAMUS)
$Z1\_6, Z2\_6, \cdots ZN\_6$

49

DEMENTIA OPINION DERIVATION UNIT

DEMENTIA OPINION DERIVATION MODEL

39

60

DEMENTIA OPINION INFORMATION

NC/MCI/AD

※NC: NORMAL CONTROL
MCI: MILD COGNITIVE IMPAIRMENT
AD: ALZHEIMER'S DISEASE

EP 4 223 229 A1

# FIG. 8

70

DIAGNOSIS SUPPORT APPLICATION

PATIENT: TARO FUJI                    IMAGING DATE: 08.24.2020  9:55

15S                    15A                    15C

71          71    ANALYSIS   72         71

# FIG. 9

60

DEMENTIA OPINION
INFORMATION

MCI

75

DIAGNOSIS SUPPORT APPLICATION

PATIENT: TARO FUJI          IMAGING DATE: 08.24.2020  9:55

15S                    15A                    15C

71          71    ANALYSIS          71
                  RESULT

⚠ SUSPECTED AS MILD
  COGNITIVE IMPAIRMENT          76

CONFIRMATION 77

28

# FIG. 10

56

ANATOMICAL REGION IMAGE

80

AE

81 — COMPRESSION UNIT

37

FEATURE AMOUNT DERIVATION MODEL

FEATURE AMOUNT SET — 58

82 — RESTORATION UNIT

RESTORATION IMAGE — 83

FIG. 11

$$k = az + by + cx + dw + ev + fu + gt + hs + ir$$

EP 4 223 229 A1

## FIG. 12

## FIG. 13

## FIG. 14

# FIG. 15

LEARNING FEATURE AMOUNT SET GROUP — 59L

CORRECT DEMENTIA OPINION INFORMATION — 60CA

90

NEURAL NETWORK/SUPPORT VECTOR MACHINE/BOOSTING

DEMENTIA OPINION DERIVATION MODEL — 39

UPDATE SETTINGS

LOSS CALCULATION

LEARNING DEMENTIA OPINION INFORMATION — 60L

EP 4 223 229 A1

## FIG. 16

( START )

ST100 ─┐

READ HEAD MRI IMAGE

ST110 ─┐

PERFORM NORMALIZATION PROCESSING OF MATCHING HEAD MRI IMAGE WITH REFERENCE HEAD MRI IMAGE

ST120 ─┐

EXTRACT PLURALITY OF ANATOMICAL REGION IMAGES OF BRAIN FROM HEAD MRI IMAGE

ST130 ─┐

INPUT ANATOMICAL REGION IMAGES TO FEATURE AMOUNT DERIVATION MODELS AND OUTPUT FEATURE AMOUNT SET FROM FEATURE AMOUNT DERIVATION MODEL

ST140 ─┐

INPUT FEATURE AMOUNT SET GROUP TO DEMENTIA OPINION DERIVATION MODEL AND OUTPUT DEMENTIA OPINION INFORMATION FROM DEMENTIA OPINION DERIVATION MODEL

ST150 ─┐

DISPLAY DEMENTIA OPINION INFORMATION

( END )

## FIG. 17

DEMENTIA OPINION INFORMATION

| DEGREE OF PROGRESS OF DEMENTIA AFTER ONE YEAR | FAST/SLOW |

~95

## FIG. 18

DEMENTIA OPINION INFORMATION

AD/DEMENTIA WITH LEWY BODY/VASCULAR DEMENTIA

~98

# FIG. 19

56 ～ ( ANATOMICAL REGION IMAGE )

100

SINGLE-TASK CNN

101 ～ COMPRESSION UNIT ⤍ ⤍ ⤍ 105 FEATURE AMOUNT DERIVATION MODEL

103 ( FEATURE AMOUNT SET )

102 ～ OUTPUT UNIT

CLASS
DEMENTIA IS DEVELOPED/DEMENTIA IS NOT DEVELOPED ～104

# FIG. 20

56L        108        104CA

( LEARNING ANATOMICAL REGION IMAGE )    ( CORRECT CLASS )

100 ～ SINGLE-TASK CNN ⟵ UPDATE SETTINGS ⟵ LOSS CALCULATION

104L
LEARNING CLASS

# FIG. 21

56 — ANATOMICAL REGION IMAGE

110

**MULTI-TASK CNN**

111 — COMPRESSION UNIT

- - - > FEATURE AMOUNT DERIVATION MODEL — 116

FEATURE AMOUNT SET — 113

112 — OUTPUT UNIT

FIRST CLASS

DEMENTIA IS DEVELOPED/DEMENTIA IS NOT DEVELOPED

114

SECOND CLASS

AGE OF PATIENT

115

# FIG. 22

56L      114CA      115CA      118

LEARNING ANATOMICAL REGION IMAGE      CORRECT FIRST CLASS      CORRECT SECOND CLASS

110    MULTI-TASK CNN  ←  UPDATE SETTINGS  ←  LOSS CALCULATION

114L    LEARNING FIRST CLASS

115L    LEARNING SECOND CLASS

EP 4 223 229 A1

FIG. 23

## FIG. 24

FEATURE AMOUNT
SET GROUP (59)

DEMENTIA OPINION DERIVATION UNIT (140)

DEMENTIA-RELATED INFORMATION

VOLUME OF HIPPOCAMPUS, SCORE OF HASEGAWA'S DEMENTIA SCALE, GENOTYPE OF ApoE GENE, AMYLOID-$\beta$ MEASUREMENT VALUE, TAU PROTEIN MEASUREMENT VALUE, APOLIPOPROTEIN MEASUREMENT VALUE, COMPLEMENT PROTEIN MEASUREMENT VALUE, TRANSTHYRETIN MEASUREMENT VALUE,···

(141)

DEMENTIA OPINION DERIVATION MODEL (142)

DEMENTIA OPINION INFORMATION (143)

EP 4 223 229 A1

## FIG. 25

59L ⟶ LEARNING FEATURE AMOUNT SET GROUP

141L ⟶ LEARNING DEMENTIA-RELATED INFORMATION

143CA ⟶ CORRECT DEMENTIA OPINION INFORMATION

148

142 ⟶ DEMENTIA OPINION DERIVATION MODEL

UPDATE SETTINGS

LOSS CALCULATION

143L ⟶ LEARNING DEMENTIA OPINION INFORMATION

EP 4 223 229 A1

## FIG. 26

ANATOMICAL REGION IMAGE — 56

COMPRESSION UNIT — 253

AE FEATURE AMOUNT SET — 255

RESTORATION UNIT — 254

RESTORATION IMAGE — 256

250

251

OUTPUT UNIT — 257

CLASS — 258

REMAIN STATE OF MCI AFTER 2 YEARS/ PROGRESS FROM MCI TO AD AFTER 2 YEARS

SINGLE-TASK CNN

FEATURE AMOUNT DERIVATION MODEL — 252

ZA — AGGREGATED FEATURE AMOUNT → TO DEMENTIA OPINION DERIVATION MODEL

EP 4 223 229 A1

## FIG. 27

FEATURE AMOUNT SET — 255

OUTPUT UNIT — 257

SA MECHANISM LAYER — 265
GAP LAYER — 266
FC LAYER — 267
SMF LAYER — 268
PCA LAYER — 269

CLASS — 258

AGGREGATED FEATURE AMOUNT — ZA

EP 4 223 229 A1

FIG. 28

EP 4 223 229 A1

## FIG. 29

FIG. 30

ZAG

AGGREGATED FEATURE AMOUNT GROUP

281

DEMENTIA-RELATED INFORMATION

280

DEMENTIA OPINION DERIVATION UNIT

DEMENTIA OPINION DERIVATION MODEL

QUANTILE NORMALIZATION UNIT

283

LINEAR DISCRIMINANT ANALYSIS UNIT

284

282

285

DEMENTIA OPINION INFORMATION

REMAIN STATE OF MCI AFTER 2 YEARS/ PROGRESS FROM MCI TO AD AFTER 2 YEARS

## FIG. 31

<span>⎯300</span>

| No. | | METHOD | LEARNING IMAGE | INPUT DATA | PREDICTION PERIOD | CORRECT RATE | AUC | SENSITIVITY | SPECIFICITY |
|---|---|---|---|---|---|---|---|---|---|
| 1 | LITERATURE A | SUPPORT VECTOR MACHINE | ADNI | HEAD MRI IMAGE, SCORE OF DEMENTIA TEST | THREE YEARS | 0.85 | − | 0.47 | 0.97 |
| 2 | LITERATURE B | RANDOM FOREST | ADNI | HEAD MRI IMAGE, GENDER, AGE | ONE YEAR | 0.74 | − | 0.77 | 0.70 |
| 3 | LITERATURE C | DEEP NEURAL NETWORK | ADNI | HEAD MRI IMAGE, PET IMAGE | THREE YEARS | 0.82 | − | 0.79 | 0.83 |
| 4 | LITERATURE D | DEEP NEURAL NETWORK | ADNI(510 + 253), Milan(23 + 27) | HEAD MRI IMAGE | THREE YEARS | 0.75 | − | 0.75 | 0.75 |
| 5 | LITERATURE E | SURVIVAL ANALYSIS | ADNI(988 + 378), AIBL(227 + 30) J−ADNI(315,104), Shimane(80,20) | HEAD MRI IMAGE, SCORE OF DEMENTIA TEST, AGE | TIME SERIES | − | 0.76 | − | − |
| 6 | LITERATURE F | MULTIMODAL DEEP LEARNING | ADNI | HEAD MRI IMAGE, SCORE OF DEMENTIA TEST, TEST RESULT OF SPINAL FLUID TEST, ATTRIBUTE DATA OF PATIENT | ONE YEAR | 0.81 | − | 0.84 | 0.80 |
| 7 | LITERATURE G | MULTIMODAL DEEP LEARNING | ADNI | HEAD MRI IMAGE, SCORE OF DEMENTIA TEST, TEST RESULT OF GENETIC TEST, GENDER, AGE | ONE YEAR | 0.86 | − | 0.82 | 0.88 |
| 8 | PRESENT EMBODIMENT (WITHOUT DEMENTIA-RELATED INFORMATION) | MULTIMODAL DEEP LEARNING | ADNI | HEAD MRI IMAGE | TWO YEARS | 0.84 | 0.93 | 0.85 | 0.84 |
| 9 | PRESENT EMBODIMENT (WITH DEMENTIA-RELATED INFORMATION) | MULTIMODAL DEEP LEARNING | ADNI | HEAD MRI IMAGE, SCORE OF DEMENTIA TEST, TEST RESULT OF GENETIC TEST, GENDER, AGE | TWO YEARS | 0.90 | 0.97 | 0.91 | 0.90 |

| INTERNATIONAL SEARCH REPORT | International application No. |
|---|---|
| | **PCT/JP2021/035194** |

**A. CLASSIFICATION OF SUBJECT MATTER**

*A61B 10/00*(2006.01)i; *G06T 7/00*(2017.01)i; *G06N 3/04*(2006.01)i; *G16H 30/20*(2018.01)i; *G16H 50/20*(2018.01)i; *G16H 50/50*(2018.01)i; *G06N 20/10*(2019.01)i; *G06N 20/20*(2019.01)i; *A61B 5/055*(2006.01)i
FI: A61B5/055 380; A61B10/00 H; G06N3/04; G06N20/10; G06N20/20; G06T7/00 350B; G06T7/00 612; G16H30/20; G16H50/20; G16H50/50

According to International Patent Classification (IPC) or to both national classification and IPC

**B. FIELDS SEARCHED**

Minimum documentation searched (classification system followed by classification symbols)

A61B10/00; G06T7/00; G06N3/04; G16H30/20; G16H50/20; G16H50/50; G06N20/10; G06N20/20; A61B5/055

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 2012/032940 A1 (HITACHI MEDICAL CORP.) 15 March 2012 (2012-03-15) paragraphs [0031]-[0129] | 1-13 |
| A | CN 110934606 A (SHANGHAI XINGMAI INFORMATION TECHNOLOGY CO., LTD.) 31 March 2020 (2020-03-31) entire text, all drawings | 1-13 |
| A | JP 2020-009186 A (CANON MEDICAL SYSTEMS CORP.) 16 January 2020 (2020-01-16) entire text, all drawings | 1-13 |
| A | US 2017/0357753 A1 (THE JOHNS HOPKINS UNIVERSITY) 14 December 2017 (2017-12-14) entire text, all drawings | 1-13 |
| A | JP 2016-202904 A (CANON KK) 08 December 2016 (2016-12-08) entire text, all drawings | 1-13 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **01 December 2021** | **21 December 2021** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (January 2015)

**INTERNATIONAL SEARCH REPORT**

| International application No. |
| --- |
| **PCT/JP2021/035194** |

**C. DOCUMENTS CONSIDERED TO BE RELEVANT**

| Category* | Citation of document, with indication, where appropriate, of the relevant passages | Relevant to claim No. |
| --- | --- | --- |
| A | JP 2015-208385 A (HITACHI, LTD.) 24 November 2015 (2015-11-24)<br>entire text, all drawings | 1-13 |
| A | JP 2011-118543 A (SHIZUOKA PREF., FUJIFILM CORP.) 16 June 2011 (2011-06-16)<br>entire text, all drawings | 1-13 |
| A | JP 2010-012176 A (HAMAMATSU PHOTONICS KK) 21 January 2010 (2010-01-21)<br>entire text, all drawings | 1-13 |

Form PCT/ISA/210 (second sheet) (January 2015)

## INTERNATIONAL SEARCH REPORT
### Information on patent family members

International application No.

**PCT/JP2021/035194**

| Patent document cited in search report | | | Publication date (day/month/year) | Patent family member(s) | | | Publication date (day/month/year) |
|---|---|---|---|---|---|---|---|
| WO | 2012/032940 | A1 | 15 March 2012 | CN 103096787 A paragraphs [0067]-[0192] | | | |
| CN | 110934606 | A | 31 March 2020 | (Family: none) | | | |
| JP | 2020-009186 | A | 16 January 2020 | (Family: none) | | | |
| US | 2017/0357753 | A1 | 14 December 2017 | (Family: none) | | | |
| JP | 2016-202904 | A | 08 December 2016 | US 2016/0306936 A1 entire text, all drawings | | | |
| JP | 2015-208385 | A | 24 November 2015 | US 2017/0042495 A1 entire text, all drawings WO 2015/163089 A1 | | | |
| JP | 2011-118543 | A | 16 June 2011 | (Family: none) | | | |
| JP | 2010-012176 | A | 21 January 2010 | US 2011/0105881 A1 entire text, all drawings WO 2010/004851 A1 KR 10-2011-0028443 A CN 102088910 A | | | |

Form PCT/ISA/210 (patent family annex) (January 2015)

## REFERENCES CITED IN THE DESCRIPTION

### Patent documents cited in the description

- JP 6483890 B

### Non-patent literature cited in the description

- **TAM, A. ; DANSEREAU, C. ; ITURRIA-MEDINA, Y ; URCHS, S. ; ORBAN, P. ; SHARMARKE, H. ; BREITNER, J.** A highly predictive signature of cognition and brain atrophy for progression to Alzheimer's dementia. *GigaScience,* 2019, vol. 8 (5), giz055
- **LEDIG, C. ; SCHUH, A. ; GUERRERO, R. ; HECKEMANN, R. A. ; RUECKERT, D.** Structural brain imaging in Alzheimer's disease and mild cognitive impairment: biomarker analysis and shared morphometry database. *Scientific reports,* 2018, vol. 8 (1), 11258
- **LU, D. ; POPURI, K. ; DING, G W. ; BALACHANDAR, R. ; BEG, M. F.** Multimodal and multiscale deep neural networks for the early diagnosis of Alzheimer's disease using structural MR and FDG-PET images. *Scientific reports,* 2018, vol. 8 (1), 5697
- **BASAIA, S. ; AGOSTA, F. ; WAGNER, L. ; CANU, E. ; MAGNANI, G ; SANTANGELO, R. ; FILIPPI, M.** Automated classification of Alzheimer's disease and mild cognitive impairment using a single MRI and deep neural networks. *NeuroImage: Clinical,* 2019, vol. 21, 101645
- **NAKAGAWA, T. ; ISHIDA, M. ; NAITO, J. ; NAGAI, A. ; YAMAGUCHI, S. ; ONODA, K.** Prediction of conversion to Alzheimer's disease using deep survival analysis of MRI images. *Brain Communications,* 2020, vol. 2 (1
- **LEE, G ; NHO, K. ; KANG, B. ; SOHN, K. A. ; KIM, D.** Predicting Alzheimer's disease progression using multi-modal deep learning approach. *Scientific reports,* 2019, vol. 9 (1), 1952
- **GOTO, T. ; WANG, C. ; LI, Y ; TSUBOSHITA, Y.** Multi-modal deep learning for predicting progression of Alzheimer's disease using bi-linear shake fusion. *Proc. SPIE 11314, Medical Imaging,* 2020